# EUROPEAN PATENT APPLICATION

(11) **EP 0 831 093 A1**
(43) Date of publication of application: **25.03.1998**
(21) Application number: 97116236.7
(22) Date of filing: 18.09.1997
(51) Int. Cl.: C07D 463/00, A61K 31/545

(54) **1-Carba-(dethia)-Cephalosporin Derivatives**

(30) Priority: 23.09.1996 EP 96115210
(71) Applicant: F. HOFFMANN-LA ROCHE AG, 4070 Basel (CH)
(72) Inventor: Angehrn, Peter, 4461 Böckten (CH); Hebeisen, Paul, 4052 Basel (CH); Heinze-Krauss, Ingrid, 79418 Schliengen (DE); Page, Malcolm, 4056 Basle (CH)
(74) Representative: Kjellsaa-Berger, Hanny, Dr.

(57) **Abstract**

1-carba-(dethia)-cephalosporin derivatives of the general formula I 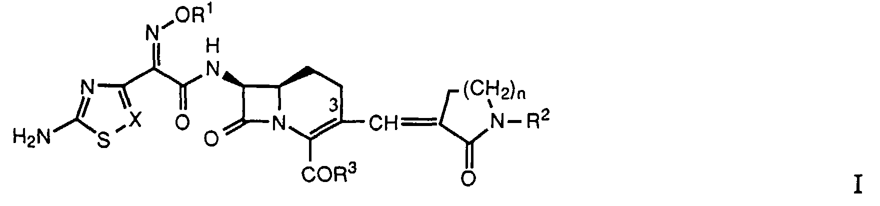 wherein
- R¹: is hydrogen, optionally fluoro substituted lower alkyl, aralkyl, cycloalkyl, -COR⁴ or -C(R⁵R⁶)CO₂R⁷ -C(R⁵R⁶)CONHR⁷; where R⁵ and R⁶ are each independently hydrogen or lower alkyl, or R⁵ and R⁶ taken together form a cycloalkyl group; R⁴ is hydrogen or lower alkyl and R⁷ is hydrogen, lower alkyl, lower alkenyl or a carboxylic acid protecting group;
- R²: is hydrogen, hydroxy, lower alkyl-Qₘ, cycloalkyl, lower alkoxy, lower alkenyl, cycloalkenyl, lower alkynyl, aralkyl-Qₘ, aryl-Qₘ, aryloxy, aralkoxy, a heterocyclic ring or heterocyclyl lower alkyl, the lower alkyl, cycloalkyl, lower alkoxy, lower alkenyl, cycloalkenyl, lower alkynyl, aralkyl, aryl, aryloxy, aralkoxy and the heterocyclic ring being unsubstituted or substituted with at least one group selected from carboxy, amino, nitro, cyano, optionally fluoro substituted lower alkyl, lower alkoxy, hydroxy, halogen, -COR⁶, -C(R⁵R⁶)CO₂R⁷, -C(R⁵R⁶)CONR⁵R⁸, -CONR⁵R⁶, -N(R⁶)COOR¹⁰, R⁶OCO- or R⁶COO- where R⁵ and R⁶ are hydrogen or lower alkyl; R⁷ is hydrogen, lower alkyl, lower alkenyl or a carboxylic acid protecting group; R⁸ is hydrogen, lower alkyl or optionally substituted phenyl; R¹⁰ is lower alkyl, lower alkenyl or a carboxylic acid protecting group;
- Q: is -CHR-, -CO- or -SO₂-;
- R: is hydrogen or lower alkyl
- R³: is hydroxy, lower-alkoxy, or -O⁻, when R² has a positive charge, or -OM and M represents an alkali metal;
- m: is 0 or 1;
- n: is 0, 1 or 2;
- X: is CH or N
as well as readily hydrolysable esters thereof, pharmaceutically acceptable salts of said compounds and hydrates of the compounds of formula I and of their esters and salts.

## Description

The present invention relates to 1-carba-(dethia)-cephalosporin derivatives of the general formula I 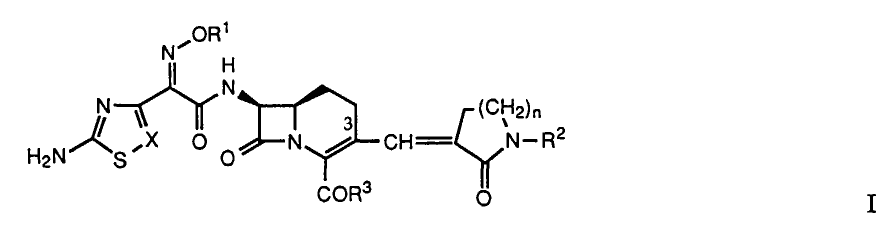 wherein
- R¹: is hydrogen, optionally fluoro substituted lower alkyl, aralkyl, cycloalkyl, -COR⁴ or -C(R⁵R⁶)CO₂R⁷ -C(R⁵R⁶)CONHR⁷; where R⁵ and R⁶ are each independently hydrogen or lower alkyl, or R⁵ and R⁶ taken together form a cycloalkyl group; R⁴ is hydrogen or lower alkyl and R⁷ is hydrogen, lower alkyl, lower alkenyl or a carboxylic acid protecting group;
- R²: is hydrogen, hydroxy, lower alkyl-Qₘ, cycloalkyl, lower alkoxy, lower alkenyl, cycloalkenyl, lower alkynyl, aralkyl-Qₘ, aryl-Qₘ, aryloxy, aralkoxy, a heterocyclic ring or heterocyclyl lower alkyl, the lower alkyl, cycloalkyl, lower alkoxy, lower alkenyl, cycloalkenyl, lower alkynyl, aralkyl, aryl, aryloxy, aralkoxy and the heterocyclic ring being unsubstituted or substituted with at least one group selected from carboxy, amino, nitro, cyano, optionally fluoro substituted lower alkyl, lower alkoxy, hydroxy, halogen, -COR⁶, -C(R⁵R⁶)CO₂R⁷, -C(R⁵R⁶)CONR⁵R⁸, -CONR⁵R⁶, -N(R⁶)COOR¹⁰, R⁶OCO- or R⁶COO- where R⁵ and R⁶ are hydrogen or lower alkyl; R⁷ is hydrogen, lower alkyl, lower alkenyl or a carboxylic acid protecting group; R⁸ is hydrogen, lower alkyl or optionally substituted phenyl; R¹⁰ is lower alkyl, lower alkenyl or a carboxylic acid protecting group;
- Q: is -CHR-, -CO- or -SO₂-;
- R: is hydrogen or lower alkyl
- R³: is hydroxy, lower-alkoxy, or -O⁻, when R² has a positive charge, or -OM and M represents an alkali metal;
- m: is 0 or 1;
- n: is 0, 1 or 2;
- X: is CH or N
as well as readily hydrolysable esters thereof, pharmaceutically acceptable salts of said compounds and hydrates of the compounds of formula I and of their esters and salts.

In above compounds of formula I the substituent in position 3 can be present in the E-form formula Ia or in the Z-form formula Ib

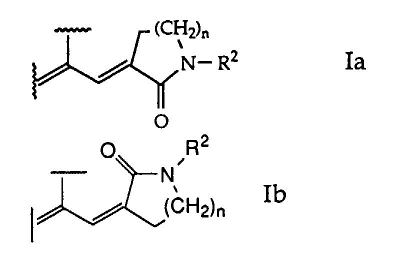

In a particular embodiment of the compounds of formula I n is 1. Moreover R¹ is preferably hydrogen, optionally fluoro substituted lower alkyl, cycloalkyl, -COR⁴, -C(R⁵R⁶)CO₂R⁷ or -C(R⁵R⁶)CONHR⁷, especially preferred are compounds of formula I wherein R¹ is hydrogen, methyl, c-pentyl, -COCH₃, -CH₂COOR⁷ or -C(CH₃)₂COOR⁷ and R⁴, R⁵ and R⁶ are as defined above whereas R⁷ is hydrogen or t-butyl.

In yet another embodiment of the compounds of formula I R² is lower alkyl-Q, where Q is -CHR- and R is hydrogen; or R² is optionally fluoro substituted lower alkyl, aryl or a heterocyclic ring, the lower alkyl, cycloalkyl, aryl, and the heterocyclic ring being unsubstituted or substituted with at least one group selected from nitro, optionally fluoro substituted lower alkyl, hydroxy or halogen.

Especially preferred compounds of formula I are compounds wherein R² respresents a pyridine ring optionally substituted in position 1, for example, pyridine-2-yl, -3-yl or -4-yl, 1-methylpridinium-2-yl, -3-yl or -4-yl, 1-carbamoylmethylpyridinium-2-yl, -3-yl or -4-yl, 1-(N-phenylcarbamoylmethyl)-pyridinium-2-yl, -3-yl or -4-yl, 1-[N-(3-fluoro-4-hydroxyphenyl)-carbamoylmethyl]pyridinium-2-yl, -3-yl or -4-yl, and the like.

The compounds of the formula I are preferably in the Z-form at the oximino group and E-form for the substitutent in position 3.

Preferred compounds of formula I include:
(6R,7S)-7-[(Z)-2-Acetoxyimino-2-(2-amino-thiazolo-4-yl)-acetylamino]-3-[(E)-1-(1-carbamoylmethyl-pyridin-1-ium-3-yl)-2-oxo-pyrrolidin-3-ylidenemethyl]-8-oxo-1-aza-bicyclo[4.2.0]oct-2-ene-2-carboxylate 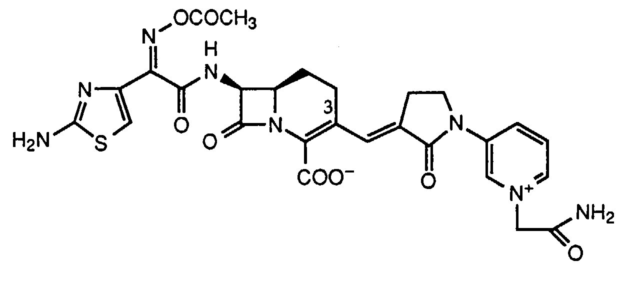
(6R,7S)-7-[(Z)-2-(2-Amino-thiazol-4-yl)-2-hydroxyimino-acetylamino]-8-oxo-3-[(E)-2-oxo-1-(2,2,2-trifluoro-ethyl)-pyrrolidin-3-ylidenemethyl]-1-aza-bicyclo[4.2.0]oct-2-ene-2-carboxylic acid sodium salt (1:1) 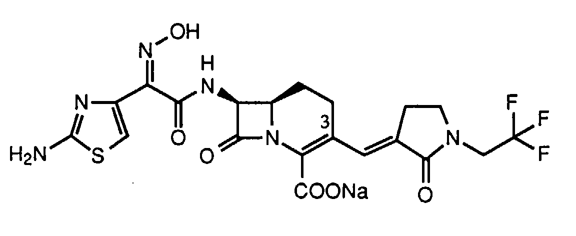
(6R,7S)-7-[(Z)-2-(Amino-thiazol-4-yl)-2-hydroxyimino-acetylamino]-3-[(E)-1-(4-hydroxy-phenyl)-2-oxo-pyrrolidin-3-ylidenemethyl]-8-oxo-1-aza-bicyclo[4.2.0]oct-2-ene-2-carboxylic acid sodium salt (1:1) 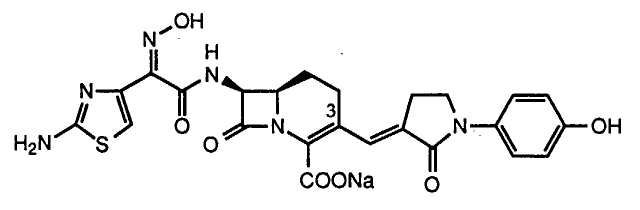
(6R,7S)-7-[(Z)-2-(2-Amino-thiazol-4-yl)-2-hydroxyimino-acetylamino]-3-[(E)-1 (1-methyl-pyridin-1-ium-3-yl)-2-oxo-pyrrolidin-3-ylidenemethyl]-8-oxo-1-aza-bicyclo[4.2.0]oct-2-ene-2-carboxylate 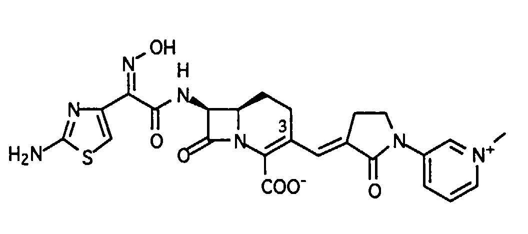
(6R,7S)-7-[(Z)-2-Acetoxyimino-2-(2-amino-thiazol-4-yl)-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate 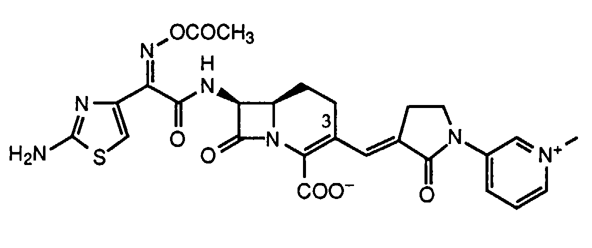
(6R,7S)-7-[(Z)-2-(2-Amino-thiazol-4-yl)-2-methoxyimino-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate 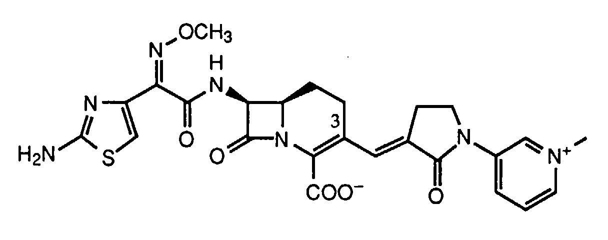
(6R,7S)-7-[(Z)-2-(2-Amino-thiazol-4-yl)-2-cyclopentyloxyimino-acetylaminol-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate 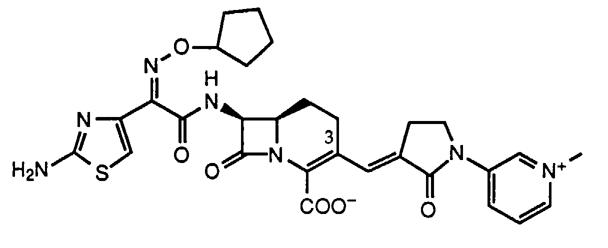
(6R,7S)-7-[(Z)-2-(5-Amino-[1,2,4]thiadiazol-3-yl)-hydroxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate 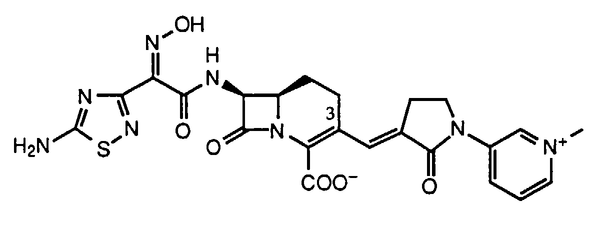
(6R,7S)-7-[(Z)-2-(5-Amino-[1,2,4]thiadiazol-3-yl)-cyclopentyloximino-acetylamino]-3[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate 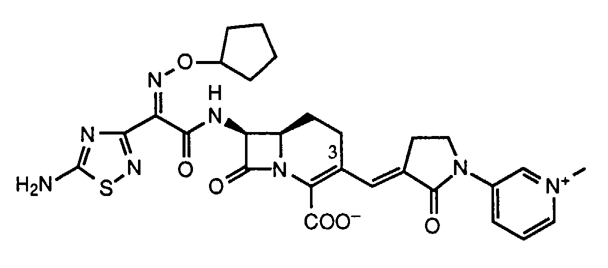
(6R,7S)-7-[(Z)-2-(2-Amino-thiazol-4-yl)-2-carboxymethoxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate Na salt (1:1) 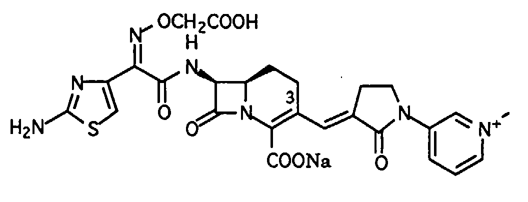
(6R,7S)-7-[(Z)-2-(2-Amino-thiazol-4-yl)-2-cyclopentyloxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate 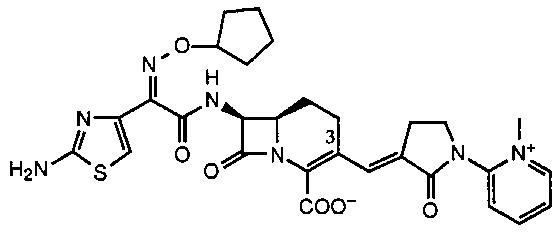
(6R,7S)-7-[(Z)-2-(2-Amino-thiazol-4-yl)-2-hydroxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate 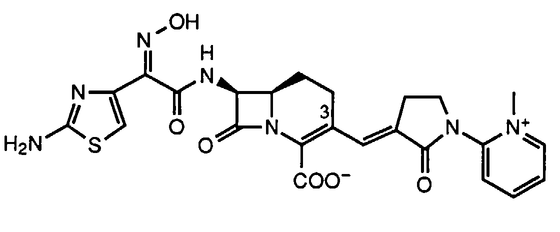
(6R,7S)-7-[(Z)-2-(2-Amino-thiazol-4-yl)-2-hydroxrimino-acetylamino]-3-[(E)-1-(1-methyl-pyridin-1-ium-4-yl)-2-oxo-pyrrolidin-3-ylidenemethyl]-8-oxo-1-aza-bicyclo[4.2.0]oct-2-ene-2-carboxylate 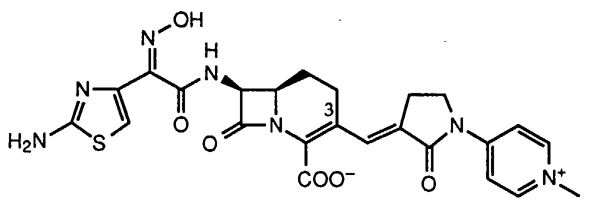
(6R,7S)-7-[(Z)-2-(2-Amino-thiazol-4-yl)-2-cyclopentyloxyimino-acetylamino]-3-[(E)-1-(1-methyl-pyridin-1-ium-4-yl)-2-oxo-pyrrolidin-3-ylidenemethyl]-8-oxo-1-aza-bicyclo[4.2.0]oct-2-ene-2-carboxylate

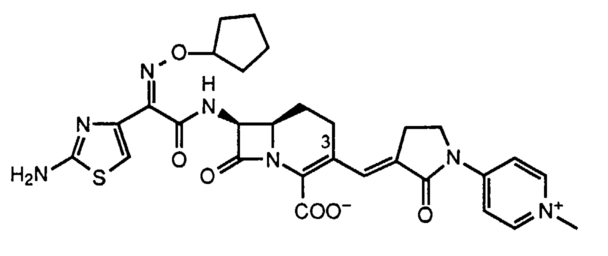

The invention also relates to pharmaceutical compositions and methods of use of the above.

As used herein, the term "optionally fluoro substituted lower alkyl" refers to both unsubstitued and fluorosubstituted straight and branched chain saturated hydrocarbon groups having 1 to 8 and preferably 1 to 4 carbon atoms, for example, methyl, ethyl, n-propyl, isopropyl, tertiary butyl which group may be mono or multiply fluoro-substituted as for example fluoromethyl, trifluoromethyl, fluoroethyl, trifluoroethyl and the like.

The term "lower alkoxy" refers to ether groups wherein alkyl is defined as above.

The term "lower alkenyl" refers to unsubstituted or substituted hydrocarbon chain radicals having from 2 to 8 carbon atoms, preferably from 2 to 4 carbon atoms, and having at least one olefinic double bond, e.g. vinyl, allyl, 1-propenyl, 1-, 2- or 3-butenyl etc.

The term "lower alkynyl" refers to unsubstituted or substituted hydrocarbon chain radicals having from 2 to 8 carbon atoms, preferably from 2 to 4 carbon atoms, and having at least one triple bond preferably located at the end of the chain, for example, acetyl?, 2-propynyl, 3-butynyl, etc.

By the term "cycloalkyl" is meant a 3-7 membered saturated carbocyclic moiety, e.g., cyclopropyl, cyclobutyl, cyclopentyl, cyclohexyl, etc.

By the term "cycloalkenyl" is meant a 3-7 membered unsaturated carbocyclic moiety, e.g. cyclopentenyl, cyclohexenyl, etc.

By the term "aralkyl" is meant an alkyl group containing an aryl group. It is a hydrocarbon group having both aromatic and aliphatic structures, that is, a hydrocarbon group in which a lower alkyl hydrogen atom is substituted by a monocyclic aryl group, e.g., phenyl, tolyl, etc.

By the term "aralkoxy" is meant an ether group wherein aralkyl is defined as above.

By the term "aryl" is meant a radical derived from an aromatic hydrocarbon by the elimination of one atom of hydrogen and can be substituted or unsubstituted. The aromatic hydrocarbon can be mononuclear or polynuclear. Examples of aryl of the mononuclear type include phenyl, tolyl, xylyl, mesityl, cumenyl, and the like. Examples of aryl of the polynuclear type include naphthyl, anthryl, phenanthryl, and the like. The aryl group can have at least one substituent selected from, as for example, halogen, hydroxy, cyano, carboxy, nitro, amino, lower alkyl, lower alkoxy, such as in 2,4-difluorophenyl, 4-carboxyphenyl, 4-nitrophenyl, 4-aminophenyl, 4-methoxyphenyl.

By the term "aryloxy" is meant an ether group wherein aryl is defined as above.

By the term "optionally substituted phenyl" is meant phenyl, or phenyl substituted with at least one of halogen, hydroxy, amino, lower alkyl or lower alkoxy, for example, phenyl, 3-fluoro-4-hydroxy-phenyl, and the like.

The term "halogen" or "halo" used herein refers to all four forms, that is chlorine or chloro; bromine or bromo; iodine or iodo; and fluorine or fluoro, unless specified otherwise.

As used herein, "heterocyclic ring" refers to an unsaturated or saturated, unsubstituted or substituted 5-, 6-, or 7-membered heterocyclic ring containing at least one hetero atom selected from the group consisting of oxygen, nitrogen, or sulfur. Exemplary heterocyclic rings include, but are not limited to, for example, the following groups: pyridyl, which is optionally substituted in position 1, for example, pyridinium-2-yl, -3-yl or -4-yl, pyrazinyl, piperidyl, piperidino, N-oxido-pyridyl, pyrimidyl, piperazinyl, pyrrolidinyl, pyridazinyl, N-oxide-pyridazinyl, pyrazolyl, triazinyl, imidazolyl, thiazolyl, 1,2,3-thiadiazolyl, 1,2,4-thiadiazolyl, 1,3,4-thiadiazolyl, 1,2,5-thiadiazolyl, 1,2,3-oxadiazolyl, 1,2,4-oxadiazolyl, 1,3,4-oxadiazolyl, 1,2,5-oxadiazolyl, 1,2,3-triazolyl, 1,2,4-triazolyl, 1H-tetrazolyl, 2H-tetrazolyl; thienyl, furyl, hexamethyleneiminyl, oxepanyl, 1H-azepinyl, thiophenyl, tetrahydrothiophenyl, 3H-1,2,3-oxathiazolyl, 1,2,3-oxadiazolyl, 1,2,5-oxadithiolyl, isoxazolyl, isothiazolyl, 4H-1,2,4-oxadiazinyl, 1,2,5-oxathiazinyl, 1,2,3,5-oxathiadiazinyl, 1,3,4-thiadiazepinyl, 1,2,5,6-oxatriazepinyl, 1,6,3,4-dioxadithiopanyl, oxazolidinyl, tetrahydrothienyl, etc. Substituents for the heterocyclic ring include, for example, lower alkyl groups such as methyl, ethyl, propyl, etc., lower alkoxy groups such as methoxy, ethoxy, etc., halogens such as fluorine, chlorine, bromine, etc., halogen substituted alkyl groups such as trifluoromethyl, trichloroethyl, etc., amino, mercapto, hydroxyl, carbamoyl, or carboxyl group. A further substituent is oxo, such as in 2-oxo-oxazolidin-3-yl, 1,1-dioxo-tetrahydrothien-3-yl. Further examples of substituted heterocycles are 1-methyl-pyridinium-2-yl, -3-yl, -4-yl, 1-ethyl-pyridinium-2-yl, -3-yl, -4-yl, 1-carbamoylmethyl-pyridinium-2-yl, -3-yl, -4-yl, 6-methoxy-pyridin-3-yl, 5-methyl-isoxazol-3-yl, 1-methyl-4-pyridinio.

The term "carboxylic acid protecting group" refers to protecting groups conventionally used to replace the acidic proton of a carboxylic acid. Examples of such groups are benzyhydryl, t-butyl, p-nitrobenzyl, p-methoxybenzyl and allyl.

As used herein pharmaceutically acceptable salts useful in this invention include salts derived from metals, the ammonium salt, quaternary ammonium salts derived from organic bases and amino acid salts. Examples of preferred metal salts are those derived from the alkali metals, for example, lithium (Li⁺), sodium (Na⁺) and potassium (K⁺) are within the scope of this invention. Examples of quaternary ammonium salts derived from organic bases include tetramethylammonium (N⁺(CH₃)₄), tetraethylammonium (N⁺(CH₂CH₃)₄), benzyltrimethylammonium (N⁺(C₆H₅CH₂)(CH₃)₃), phenyltriethylammonium (N⁺(C₆H₅)(CH₂CH₃)₃), and the like, etc. Those salts derived from amines include salts with N-ethylpiperidine, procaine, dibenzylamine, N,N'-dibenzylethylenediamine, alkylamines or dialkylamines as well as salts with amino acids such as, for example, salts with arginine or lysine. Especially prefered are hydrochlorides, sulfates, phosphates, lactates, mesylates or the inner salt.

As readily hydrolysable esters of the compounds of formula I there are to be understood compounds of formula I, the carboxy group(s) of which (for example, the 2-carboxy group) is/are present in the form of readily hydrolysable ester groups. Examples of such esters, which can be of the conventional type, are the lower alkanoyloxy-alkyl esters (e.g., the acetoxymethyl, pivaloyloxymethyl, 1-acetoxyethyl and 1-pivaloyloxyethyl ester), the lower alkoxycarbonyloxyalkyl esters (e.g., the methoxycarbonyloxymethyl, 1-ethoxycarbonyloxyethyl and 1-isopropoxycarbonyloxyethyl ester), the lactonyl esters (e.g., the phthalidyl and thiophthalidyl ester), the lower alkoxymethyl esters (e.g., the methoxymethyl ester) and the lower alkanoylaminomethyl esters (e.g., the acetamidomethyl ester). Other esters (e.g., the benzyl and cyanomethyl esters) can also be used. Other examples of such esters are the following: (2,2-dimethyl-1-oxopropoxy)methyl ester; 2-[(2-methylpropoxy)carbonyl]-2-pentenyl ester; 1-[[(1-methylethoxy)carbonyl]oxy] ethyl ester; 1-(acetyloxy) ethyl ester; (5-methyl-2-oxo-1,3-dioxol-4-yl) methyl ester; 1-[[(cyclohexyloxy)carbonyl]oxy] ethyl ester; and 3,3-dimethyl-2-oxobutyl ester. It will be appreciated by those of ordinary skill in the art that the readily hydrolysable esters of the compounds of the present invention can be formed at a free carboxy group of the compound, for example, at the carboxy group in position 1 and at a carboxy group -COOR⁷.

The compounds of formula I as well as their salts and readily hydrolysable esters can be hydrated. The hydration can be effected in the course of the manufacturing process or can occur gradually as a result of hygroscopic properties of an initially anhydrous product.

The compounds of the present invention are useful as antibiotics having potent and broad antibacterial activity; especially against methicillinresistent staphylococci (MRSA) and *Pseudomonas aeruginosa*.

The products in accordance with the invention can be used as medicaments, for example, in the form of pharmaceutical preparations for parenteral administration, and for this purpose are preferably made into preparations as lyophilisates or dry powders for dilution with customary agents, such as water or isotonic common salt solution.

Depending on the nature of the pharmacologically active compound the pharmaceutical preparations can contain the compound for the prevention and treatment of infectious diseases in mammals, human and non-human, a daily dosage of about 10 mg to about 4000 mg, especially about 50 mg to about 3000 mg, is usual, with those of ordinary skill in the art appreciating that the dosage will depend also upon the age, conditions of the mammals, and the kind of diseases being prevented or treated. The daily dosage can be administered in a single dose or can be divided over several doses. An average single dose of about 50 mg, 100 mg, 250 mg, 500 mg, 1000 mg, and 2000 mg can be contemplated.

Representative compounds of the present invention were tested.

In vitro activity was determined by minimum inhibitory concentration in a microorganism spectum by the agar dilution method in Mueller Hinton agar, inoculum = 10⁴ CFU/spot.

The following compounds were tested:
A : (6R,7S)-7-[(Z)-2-(2-Amino-thiazol-4-yl)-2-hydroxyimino-acetylamino]-8-oxo-3-[(E)-2-oxo-1-(2,2,2-trifluoro-ethyl)-pyrrolidin-3-ylidenemethyl]-1-aza-bicyclo[4.2.0]oct-2-ene-2-carboxylic acid sodium salt (1:1)
B: (6R,7S)-7-[(Z)-2-(Amino-thiazol-4-yl)-2-hydroxyimino-acetylamino]-3-[(E)-1-(4-hydroxy-phenyl)-2-oxo-pyrrolidin-3-ylidenemethyl]-8-oxo-1-aza-bicyclo[4.2.0]oct-2-ene-2-carboxylic acid sodium salt (1:1)
C: (6R,7S)-7-[(Z)-2-(2-Amino-thiazol-4-yl)-2-hydroxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate
D: (6R,7S)-7-[(Z)-2-(5-Amino-[1,2,4]thiadiazol-3-yl)-hydroxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate
E: (6R,7S)-7-[(Z)-2-(5-Amino-[1,2,4]thiadiazol-3-yl)-cyclopentyloximino-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate
F: (6R,7S)-7-[(Z)-2-(2-Amino-thiazol-4-yl)-2-carboxymethoxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate Na salt (1:1) and
G: (6R,7S)-7-[(Z)-2-(2-Amino-thiazol-4-yl)-2-cyclopentyloxyimino-acetylamino]-3-[(E)-1-(1-methyl-pyridin-1-ium-4-yl)-2-oxo-pyrrolidin-3-ylidenemethyl]-8-oxo-1-aza-bicyclo[4.2.0]oct-2-ene-2-carboxylate

The antibacterial Spectrum appears below:
MIC : Minimum Inhibiting Concentration Values

| **Antibacterial Spectrum (MIC,** µ**g/ml)** | | | | | | | |
|---|---|---|---|---|---|---|---|
| | A | B | C | D | E | F | G |
| *S*.*aureus* 6538 | 1 | 1 | 1 | 0.5 | 0.5 | 32 | 2 |
| *S*.*aureus* 743 (MRSA) | 32 | 32 | 8 | 8 | 4 | >32 | 32 |
| *S*.*pyogenes* b 15 | ≤ 0.06 | ≤ 0.06 | ≤ 0.06 | ≤ 0.06 | ≤ 0.06 | 0.12 | ≤ 0.06 |
| *S*.*pneumoniae*. 907 | ≤ 0.06 | ≤ 0.06 | ≤ 0.06 | ≤ 0.06 | ≤ 0.06 | ≤ 0.06 | ≤ 0.06 |
| *E*.*faecalis* 6 | 8 | 4 | 0.5 | 0.25 | 1 | >32 | 1 |
| *E*.*coli* 25922 | ≤ 0.06 | 0.25 | ≤ 0.06 | 0.06 | ≤ 0.06 | ≤ 0.06 | ≤ 0.06 |
| *E*.*coli* TEM1 | 0.12 | 0.12 | ≤ 0.06 | 0.25 | 1 | ≤ 0.06 | 0.5 |
| *K*.*pneumoniae* 418 | 0.12 | 0.12 | ≤ 0.06 | ≤ 0.06 | 0.25 | ≤ 0.06 | 0.25 |
| *S*.*marcescens* 69438 | 2 | 4 | 0.12 | 0.12 | 1 | ≤ 0.06 | 0.5 |
| *E*.*cloacae* 908SSi | 2 | 0.5 | ≤ 0.06 | 0.12 | ≤ 0.06 | ≤ 0.06 | ≤ 0.06 |
| *E*.*cloacae* 908R | >32 | >32 | 2 | 1 | 0.5 | 4 | 2 |
| *C*.*freundii* 902 | 0.25 | 0.5 | ≤ 0.06 | ≤ 0.06 | ≤ 0.06 | ≤ 0.06 | ≤ 0.06 |
| *C*.*freundii* 43 | 8 | 32 | 0.5 | 0.5 | 0.5 | 2 | 1 |
| *P*.*mirabilis* 2117 | ≤ 0.06 | - | ≤ 0.06 | 0.12 | 2 | ≤ 0.06 | 1 |
| *P*.*vulgaris* 1028 | >32 | >32 | 16 | >32 | 32 | 0.12 | 16 |
| *P*.*aeruginosa* ATCC27853 | 32 | 32 | 4 | 8 | 8 | 1 | 4 |

The compounds of the formula I in accordance with the invention as well as their pharmaceutical acceptable salts, hydrates, or readily hydrolyzable esters can be manufactured in accordance with the invention by
(a) treating a compound having the formula II 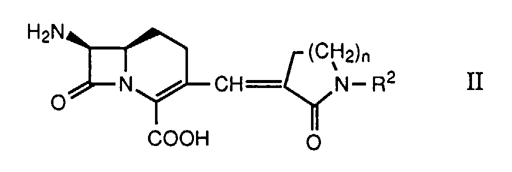 in which R² and n are defined above,
   or an ester or salt thereof, with a carboxylic acid of the general formula III 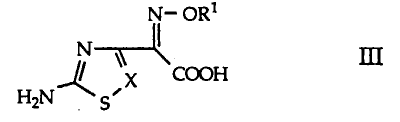 in which R¹ and X are defined above, or a reactive functional derivative thereof, or
(b) cleaving off the amino, hydroxy and/or carboxy protecting group in a compound having the formula IV 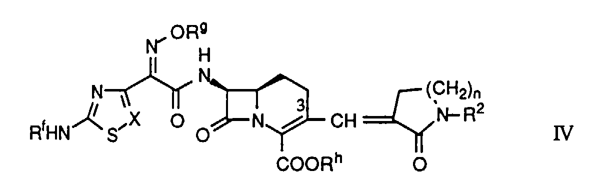 in which R² is defined above, R^{f} is hydrogen or an amino protecting group, R^{g} is hydrogen or a hydroxy protecting group, R^{h} is hydrogen or a carboxy protecting group, provided that at least one of R^{f}, R^{g} and R^{h} is a corresponding protecting group or a salt thereof, or by
(c) alkylation of a compound of formula 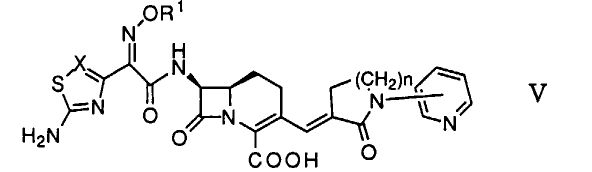 wherein R¹, X and n are as defined above,
   with a alkylating agent such a methyliodide, dimethylsulfate, trimethyloxonium tetrafluoroborate, bromo- or iodoacetamide, or
(d) for the manufacture of a readily hydrolysable ester of a compound of formula I subjecting a carboxylic acid of formula I to a corresponding esterification, or
(e) for the manufacture of salts or hydrates of a compound of formula I or hydrates of said salts converting a compound of formula I into a salt or hydrate or into a hydrate of said salts.

The reaction of compounds of formula II and III or a reactive derivative of of formula III according to embodiment (a) can be carried out in a manner known per se. The carboxy group in compounds of formula II can be protected; for example, by esterification to form a readily cleavable ester such as a silyl ester (e.g. the trimethylsilyl ester) or benzhydryl ester. The carboxy group can also be protected in the form of one of the aforementioned readily hydrolysable esters. Furthermore, the carboxy group can be protected by salt formation with an inorganic or tertiary organic base such as triethylamine. Possible protecting groups are e.g. benzhydryl, tert. butyl, p-nitro-benzyl, p-methoxy-benzyl or allyl.

The amino group present in the acylating agent of formula III can be protected. Possible protecting groups R^{f} are, for example, protecting groups which are cleavable by acid hydrolysis (e.g. the tert.butoxycarbonyl or trityl groups) or by basic hydrolysis (e.g. the trifluoroacetyl group). Preferred protecting groups are the phenylacetyl, the chloroacetyl, bromoacetyl and iodoacetyl groups, especially the chloroacetyl group. These last-mentioned protecting groups can be cleaved off by treatment with thiourea. The 7-amino group in compounds II can be protected, for example, by a silyl protecting group such as the trimethylsilyl group.

In reacting a 7-amino compound of formula II with a carboxylic acid of formula III or a reactive functional derivative thereof, for example, a free carboxylic acid can be reacted with an aforementioned ester of a compound of formula II in the presence of a carbodiimide such as dicyclohexylcarbodiimide in an inert solvent such as ethyl acetate, acetonitrile, dioxan, chloroform, methylene chloride, benzene or dimethylformamide, and subsequently the ester group can be cleaved off. Oxazolium salts (e.g. N-ethyl-5-phenyl-isoxazolium-3'-sulphonate) can be used in place of carbodiimides in the foregoing reaction.

According to another embodiment, a salt of an acid of formula II (e.g. a trialkylammonium salt such as the triethylammonium salt) is reacted with a reactive functional derivative of a carboxylic acid of formula III as mentioned earlier in an inert solvent (e.g. one of the aforementioned solvents).

According to a further embodiment, an acid halide, preferably the chloride, of a carboxylic acid of formula III is reacted with an amine of formula II. The reaction is preferably carried out in the presence of an acid-binding agent, for example in the presence of aqueous alkali, preferably sodium hydroxide, or in the presence of an alkali metal carbonate such as potassium carbonate or in the presence of a lower alkylamine such as triethylamine. As the solvent there is preferably used water, optionally in admixture with an inert organic solvent such as tetrahydrofuran or dioxan. The reaction can also be carried out in an aprotic organic solvent such as dimethylformamide, dimethylacetamide, dimethylsulphoxide or hexamethylphosphoric acid triamide. When a silylated compound of formula II is used, the reaction is carried out in an anhydrous medium.

Advantageous alternatives for acylation, where the amino group present in the acylating agent of formula III need not be protected, involves the use of a 2-benzothiazolyl thioester, a 1-hydroxybenzotriazole ester or a mixed anhydride of thiophosphoric acid of the carboxylic acid. For instance, the 2-benzthiazolyl thioester may be reacted with the compound II in an inert organic solvent such as a chlorinated hydrocarbon e.g. methylene chloride, in acetone, ethyl acetate or in a mixture of such solvents with water. The 1-hydroxybenzotriazole ester can be employed by reacting the carboxylic acid with 1-hydroxybenzotriazole and a carbodiimide, especially N,N'-dicyclohexylcarbodiimide or N,N'-diisopropylcarbodiimide in an inert organic solvent, preferably methylene chloride, dimethylformamide, tetrahydrofuran, acetonitrile or ethyl acetate.

The reaction of a 7-amino compond of formula II with the carboxylic acidof formula III or a reactive derivative thereof can conveniently be carried out at a temperature between about -40°C and +60°C, e.g. at room temperature.

Embodiment (b) of the process of the present invention involves deprotection (removal) of protected amino, hydroxy or carboxylic groups present in a compound of formula IV and can be carried and as follows:

### Removal of amino protecting groups

Possible amino-protecting groups are those employed in peptide chemistry, such as an alkoxycarbonyl group, e.g., t-butoxycarbonyl, etc., a substituted alkoxycarbonyl group, e.g., trichloroethoxycarbonyl etc., an optionally substituted aralkyloxycarbonyl group, e.g., p-nitrobenzyloxycarbonyl or benzyloxycarbonyl, an aralkyl group such as trityl or benazhydryl or a halogen-alkanoyl group such as chloroacetyl, bromoacetyl, iodoacetyl or trifluoroacetyl.

Preferred protecting groups are t-butoxycarbonyl (t-BOC) and trityl.

The amino protecting groups may be cleaved off by acid hydrolysis (e.g. the t-butoxycarbonyl or trityl group), e.g. aqueous formic acid, or by basic hydrolysis (e.g. the trifluoroacetyl group). The chloroacetyl, bromoacetyl and iodoacetyl groups are cleaved off by treatment with thiourea.

Amino-protecting groups which are cleavable by acid hydrolysis are preferably removed with the aid of a lower alkanecarboxylic acid which may be halogenated. In particular, formic acid or trifluoroacetic acid is used. The reaction is carried out in the acid or in the presence of a co-solvent such as a halogenated lower alkane, e.g. methylene chloride. The acid hydrolysis is generally carried out at room temperature, although it can be carried out at a slightly higher or slightly lower temperature (e.g. a temperature in the range of about -30°C to +40°C). Protecting groups which are cleavable under basic conditions are generally hydrolyzed with dilute aqueous caustic alkali at 0°C to 30°C. The chloroacetyl, bromoacetyl and iodoacetyl protecting groups can be cleaved off using thiourea in acidic, neutral or alkaline medium at about 0°C-30°C.

### Removal of hydroxy protecting groups

Possible hydroxy protecting groups are such as are commonly known in the art, e.g.
- for protection of hydroxyimino groups (R¹ = hydrogen in compounds of formula I), usually trityl, lower alkanoyl, preferably acetyl, tetrahydropyranyl protecting groups are employed.

These protecting groups are e.g. removed as follows:
- -trityl: in acidic solvents like 90% formic acid at about 0 to 50°C or triethylsilane in trifluoroacetic acid at about -20 to 25°C;
in organic solutions of hydrochloric acid at about -50 to 25°C;
- -acetyl: with weak inorganic bases like sodium bicarbonate in ethanol/water at about 0 to 50°C;
- -tetrahydropyranyl: with weak organic acids like p-toluenesulfonic acid in an alcohol, e.g. ethanol, at about 0°C to the boiling point of the mixture.

### Removal of protecting groups at the carboxy function

As ester protecting groups one may utilize an ester form which can be easily converted into a free carboxyl group under mild conditions, the ester protecting group being exemplified by, for example, t-butyl, p-nitrobenzyl, p-methoxybenzyl, benzhydryl, allyl, etc.

These protecting groups may be removed as follows:
- benzhydryl: trifluoroacetic acid with anisol, phenol, cresol or triethylsilane at about -40°C to room temperature; hydrogen with Pd/C in an alcohol such as ethanol or in tetrahydrofuran; BF₃-etherate in acetic acid at about 0 to 50°C;
- t-butyl: formic acid or trifluoroacetic acid with or without anisol, phenol, cresol or triethylsilane and a solvent such as dichloromethane at about -10°C to room temperature;
- p-nitrobenzyl: sodium sulfide in acetone/water at about 0 to room temperature; or hydrogen with Pd/C in an alcohol such as ethanol or in tetrahydrofuran;
- p-methoxybenzyl: formic acid at about 0 to 50°C; or trifluoroacetic acid and anisol, phenol or triethylsilane at about -40°C to room temperature;
- allyl: palladium(O) catalyzed transalkylation reaction in the presence of sodium or potassium salt of 2-ethyl hexanoic acid, see for example J. Org. Chem. 1982, 47, 587.

In order to manufacture a readily hydrolysable ester of the carboxylic acids of formula I in accordance with embodiment (c) of the process provided by the present invention, a carboxylic acid of formula I is preferably reacted with a corresponding halide, preferably an iodide, containing the desired ester group. The reaction can be accelerated with the aid of a base such as an alkali metal hydroxide, an alkali metal carbonate or an organic amine such as triethylamine. The esterification is preferably carried out in an inert organic solvent such as dimethylacetamide, hexamethylphosphoric acid triamide, dimethyl sulfoxide or, especially, dimethylformamide. The reaction is preferably carried out at a temperature in the range of about 0-40°C.

The manufacture of the salts and hydrates of the compounds of formula I or the hydrates of said salts in accordance with embodiment (d) of the process provided by the present invention can be carried out in a manner known per se; for example, by reacting a carboxylic acid of formula I or a salt thereof with an equivalent amount of the desired base, conveniently in a solvent such as water or an organic solvent (e.g. ethanol, methanol, acetone and the like). Correspondingly, salt formation is brought about by the addition of an organic or inorganic salt. The temperature at which the salt formation is carried out is not critical. The salt formation is generally carried out at room temperature, but it can be carried out at a temperature slightly above or below room temperature, for example in the range of 0°C to +50°C.

The manufacture of the hydrates usually takes place automatically in the course of the manufacturing process or as a result of the hygroscopic properties of an initially anhydrous product. For the controlled manufacture of a hydrate, a completely or partially anhydrous carboxylic acid of formula I or salt thereof can be exposed to a moist atmosphere (e.g. at about +10°C to +40°C).

Exemplary of the process for obtaining products in accordance with the invention are the following reaction schemes 1 and 2 below.

### Scheme 1

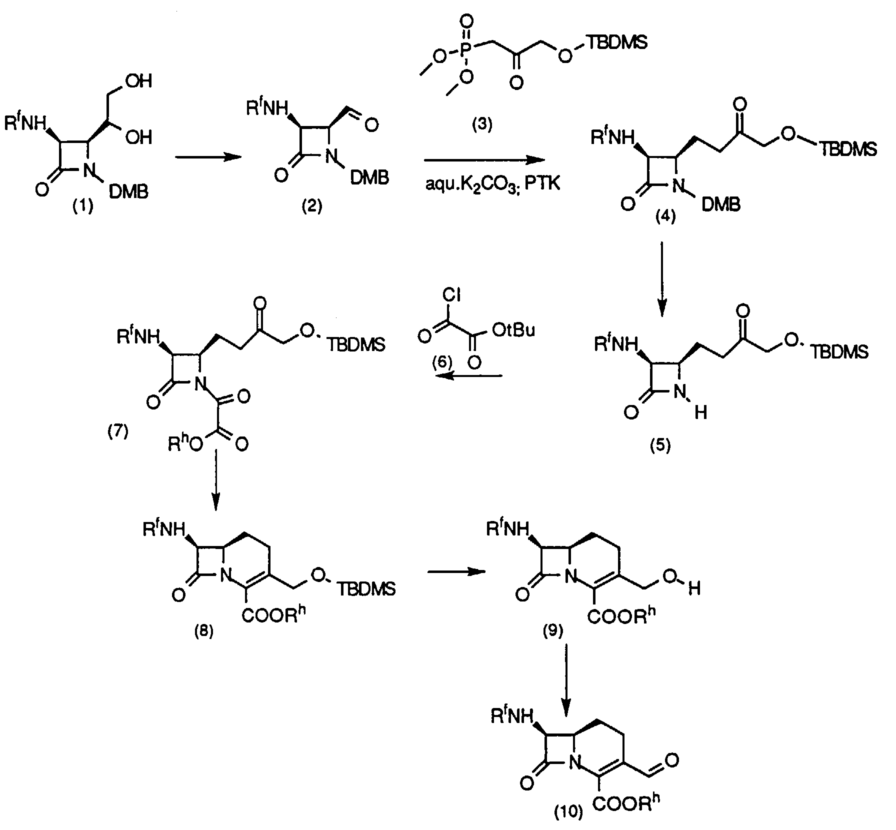

wherein R^{f} is an amino protecting group as defined above, R^{h} is an carboxy protecting group as defined above, PTK stands for phase transfer catalyst, and TBDMS is the tert.butyl-dimethylsilanyloxy group.

### (1)→(2)

The 2-(1,2-dihydroxyethyl)-4-oxo-azetidine derivative (1) is oxidized by a standard method to form the aldehyde (2). The oxidation is preferably performed with sodium periodate in an inert solvent as e.g. tetrahydrofuran/water.

### (2)→(4)

The aldehyde (2) is reacted with the phosphonic acid ester (3) in the presence of a base, preferably potassium carbonate and of a phase transfer catalyst. The unsaturated addition product is subsequently hydrogenated to form compound (4). The hydrogenation of the double bond is preferably carried out in presence of an catalytic amount of Pd on charcoal in an inert solvent as e.g. ethylacetate.

### (4)→(5)

Deprotection of the azetidinone-nitrogen in compound (4) is carried out in a manner known per se, preferably by addition of potassium persulfate to a solution of (4) in acetonitril, during the reaction the pH of the solution is preferably about 5.

### (5)→(7)

After deprotection the azetidinone-nitrogen of (5) is acylated with an apropriate 2-oxo-acetic acid derivative, preferably with 2-chloro-2-oxo-acetic acid tert.butylester (6).

### (7)→(8)

Cyclisation of compound (7) is preferably carried out in presence of an alkylphosphit and optionally a radical scavenger as for example hydroquinone to form compound (8).

### (8)→(9)→(10)

The selective deprotection of the hydroxy group in (8) and the subsequent oxidation of this group to form the aldehyd (10) is carried out according to methods known in the art. Preferred oxidation agents are pyridinium dichromate, manganese dioxide, or sodium hypochlorite in presence of catalytic amounts of piperidin-1-oxyl radicals as TEMPO (2,2,6,6-tetramethylpiperidin-1-oxyl radical) or conditions used for SWERN oxidation.

### Scheme 2

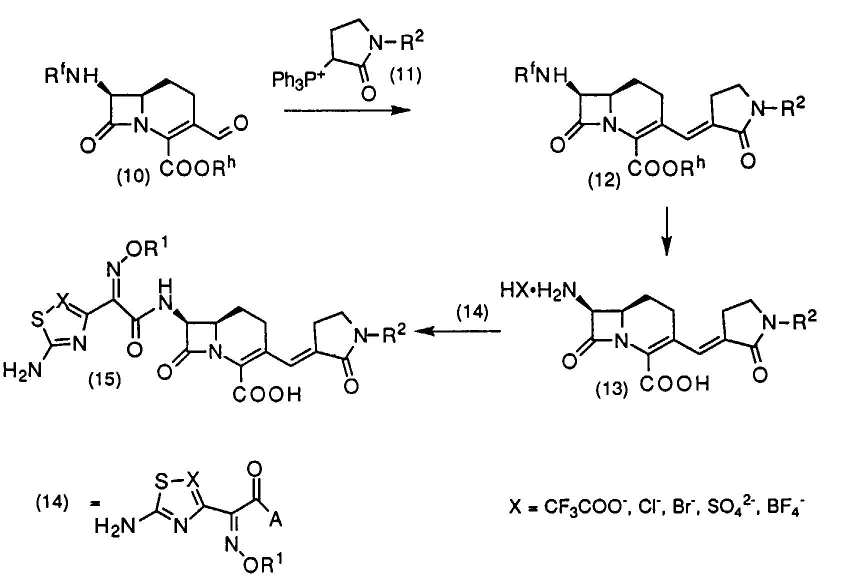

wherein A is an activating group as described below.

### (10)→(12)

The reaction of 2-carba-(dethio)-cephem aldehyde (10) where R^{h} is a carboxy protecting group as defined above, e.g. benzhydryl ester, and R^{f} is an amino protecting group as defined above, e.g. tert. butyloxycarbonyl, with a Wittig reagent, exemplified by structure (11), yields the coupling product (12). The reaction is carried out in the presence of a base which is either an inorganic base (sodium or potassium hydroxide, sodium or potassium carbonate etc.), an organic base (tertiary amines), an organolithium such as butyl lithium or phenyllithium or an epoxide such as 1,2-butyleneoxide. The reaction in presence of an epoxide is preferred. The preferred solvents, in the case of inorganic base being used, are water and water-miscible solvent (acetone, tetrahydrofuran, or alcohols etc.); in the case of organic base being used, an inert solvent such as methylene chloride, chloroform, benzene, tetrahydrofuran; in the case of organolithium being used, benzene or tetrahydrofuran; and in the case an epoxide being used, the epoxide itself (e.g. 1,2-butyleneoxide). The temperature for the reaction ranges from -20°C to 110°C. The preferred conditions are exemplified in the examples.

In the normal Wittig reaction according to scheme 2, the E isomer is the predominant product. Invariably, less than 10% Z-isomer is formed, the amount depending on the reagents and conditions.

### (12)→(13)

The protecting groups R^{f} and R^{h} are removed and the reaction conditions used are depending on the nature of the protecting groups. In the case of R^{f} being tert-butoxycarbonyl and R^{h} being benzhydryl, trifluoroacetic acid and anisole or triethylsilane is employed, at temperature of about -20°C to about room temperature (about 22°C).

### (13)→(15)

The acylation of compound (13) can be carried out with an organic acid (14) which is activated with known reagents A, preferably thionyl chloride, oxalyl chloride, dicyclohexylcarbodiimide, bis-[benzthiazolyl-(2)]disulfide, N-hydroxy-benzotriazole, a 2-halo N-methylpyridinium salt or a mixed anhydride of thiophosphoric acid e.g. of diethylthiophosphoric acid. The reaction is carried out with or without the base (inorganic or organic bases) depending on the method of activation and a wide range of solvents, from water and water-miscible solvent to inert solvents such as chloroform, dimethylformamide (DMF) or dimethylsulfoxide (DMSO) can be used. The substituents of R¹ group, if necessary, can be further deprotected with a reaction condition suitable for the removal of the protecting group.

For the preparation of optionally substituted pyridinium derivatives, i.e. compounds of formula I wherein R² represent a pyridinium residue the quaternisation of the corresponding pyridine derivative can either be performed subsequent to the Wittig reaction (10)→(12) by substituting compounds (12) wherein R² is 2-, 3- or 4-pyridinyl in presence of an alkylating agent as for example methyliodide, dimethylsulfate or trimethyloxonium tetrefluoroborate, bromo- or iodoacetamide in a suitable solvent which may be chosen from N,N-dimethylformamide or the like; or the quaternisation can be performed subsequent to the acylation step (13)→ (**15**) under the conditions named above, however, alkylation at this step requires intermediate protection of the other sensitive groups in compound (**15**). The intermediate protection of these group is preferably carried out with bis(trimethylsilyl)acetamide, see scheme 3.

### Scheme 3

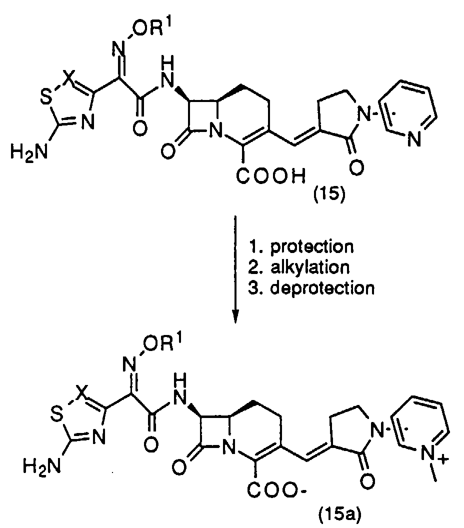

Preferably the quaternisation of the pyridine ring is performed subsequent to the Wittig reaction.

### Example 1

### Synthesis of [(2S,3S)-1-(2,4-Dimethoxy-benzyl)-2-formyl-4-oxo-azetidin-3-yl]-carbamic acid tert-butyl ester (2)

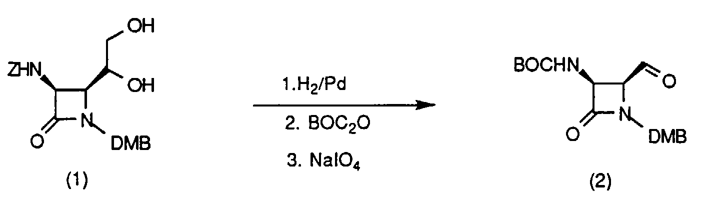

wherein Z is benzyl, DMB is dimethoxybenzyl and BOC is tert.butyloxycarbonyl.
To a solution of 975 g (2.25 Mol) [(2S,3S)-2-[(R)-1,2-dihydroxy-ethyl]-1-(2,4-dimethoxy-benzyl)-4-oxo-azetidin-3-yl]-carbamic acid benzyl ester (1) in 9.00 l tetrahydrofurane and 1.875 l water is added 112 g palladium 5% on charcoal and the mixture is stirred under a hydrogen atmosphere at 19-22°C for 2.5 h. The catalyst is removed by filtration and washed with a mixture of 0.75 l tetrahydrofurane and 0.15 l water. To the combined filtrate is added 540 g (2.475 Mol) di-*tert*-butyl dicarbonate and the mixture is stirred at 23-30°C for 17 h. A solution of 530 g (2.478 Mol) sodium periodate in 4.05 l water is added at 26-30°C during 15 min. and stirring is continued for 4 h at 28-30°C. The mixture is diluted with 8.5 l water and 7.5 l ethyl acetate. The phases are separated, the organic phase is washed with 6.75 l of a 10% solution of sodium bicarbonate and with 6.0 l of brine. The aqueous phases are reextracted with 3.7 l of ethyl acetate. The combined organic phases are dried over 2000 g sodium sulfate, filtered and the solids are washed with 2.0 l of ethyl acetate. The combined filtrates are concentrated under aspirator vacuum to yield 940 g of a yellow foam. This foam is dissolved in 1.9 l methylene chloride at 40°C, diluted with 4.5 l n-hexane, concentrated to 4.3 l at 40°C and 600 mbar and allowed to crystallize for two days. The resulting crystals are collected by filtration, washed with 2.5 l n-hexane and dried under aspirator vacuum at 40°C for 20 h to yield 816 g [(2S,3S)-1-(2,4-dimethoxy-benzyl)-2-formyl-4-oxo-azetidin-3-yl]-carbamic acid tert-butyl ester (2) as colourless crystals (97.2% Th.); m.p.: 128-131°C
¹H-NMR (CDCl₃): 1.40 (s,9H); 3.76 (s,3H); 3.80 (s,3H); 4.12 (d,J=6Hz,1H); 4.34 (d,J=14Hz, 1H); 4.58 (d,J=14Hz, 1H); 4.87 (dd,J=8Hz,J=6Hz,1H); 5.10 (d,J=8Hz,1H) 6.43 (m,2H); 7.12 (m,1H); 9.55 (s,1H) ppm.

### Example 2

### Synthesis of (2R,3S)-[2-[4-(tert-Butyl-dimethyl-silanyloxy)-3-oxo-butyl]-1-(2,4-dimethoxy-benzyl)-4-oxo-azetidin-3-yl]-carbamic acid tert-butyl ester(4)

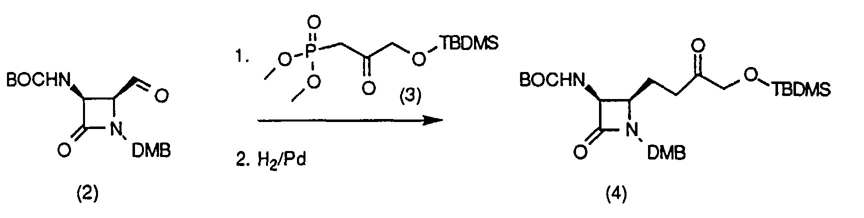

wherein TBDMS is tert.butyl-dimethyl-silanyloxy and the remaining symbols are as defined above.
To a solution of 501 g (1.37 Mol) [(2S,3S)-1-(2,4-dimethoxy-benzyl)-2-formyl-4-oxo-azetidin-3-yl]-carbamic acid tert-butyl ester (2) in 4.0 l ethyl acetate is added at 0-5°C 534 g (1.66 Mol) [3-(tert-butyl-dimethyl-silanyloxy)-2-oxo-propyl]-phosphonic acid dimethyl ester (3) and a solution of 303 g (2.2 Mol) potassium carbonate and 47 g (0.14 Mol) tetrabutylammonium hydrogen sulfate in 1.03 l water. This mixture is stirred at 0-5°C for 3 h. The phases are separated and the organic phase is washed 3 times with 460 ml of a 10 % solution of sodium chloride. The aqueous phases are extracted with 400 ml ethyl acetate. The combined organic phases are dried over 600 g sodium sulfate, filtered, the solids are washed with 200 ml ethyl acetate and the combined filtrates are stirred over 50 g palladium 5% on charcoal under a hydrogen atmosphere at normal pressure and room temperature for 5 h. The catalyst is removed by filtration, the solids are washed with 500 ml ethyl acetate. The combined filtrates are concentrated under aspirator vacuum at 40°C to yield 961 g of a yellow oil. This oil is taken up in ethyl acetate : n-hexane = 1 : 3 and filtered over 2.5 kg of silica gel 60 (230-400 mesh) with ethyl acetate : n-hexane = 1: 3 as eluent. The fractions pure product containing are combined and concentrated under aspirator vacuum to yield 684 g (2R,3S)-[2-[4-(tert-butyl-dimethyl-silanyloxy)-3-oxo-butyl]-1-(2,4-dimethoxy-benzyl)-4-oxo-azetidin-3-yl]-carbamic acid tert-butyl ester (4) as a yellow amorphous solid (90.6% Th.).
¹H-NMR (DMSO): δ 0.01 (s,6H); 0.84 (s,9H); 1.38 (s,9H); 1.56 (m,1H); 1.75 (m,1H); 2.23 (m,2H); 3.42 (m,1H); 3.74 (s,3H); 3.77 (s,3H); 4.05 (d,J=15Hz,1H); 4.15 (s,2H); 4.26 (d,J=15Hz,1H); 4.75 (dd,J=10Hz,J=5Hz,1H); 6.46 (dd,J=8Hz,J=2Hz,1H); 6.55 (d,J=2Hz,1H); 7.13 (d,J=8Hz,1H); 7.78 (d,J=10Hz,1H) ppm.
MS (ISP): 537.3 (M+H⁺), 559.3 (M+Na⁺).
IR (KBr): 1455 cm⁻¹(ν β-Lactam CO)

| | | | |
|---|---|---|---|
| M A calc.for C₂₇H₄₄N₂O₇Si: | C:60.42; | H:8.26; | N:5.22; |
| found: | C:60.19; | H:8.30; | N:5.16 (%) |

### Example 3

### Synthesis of (2R,3S)-[2-[4-(tert-Butyl-dimethyl-silanyloxy)-3-oxo-butyl]-4-oxo-azetidin-3-yl]-carbamic acid tert-butyl ester (5)

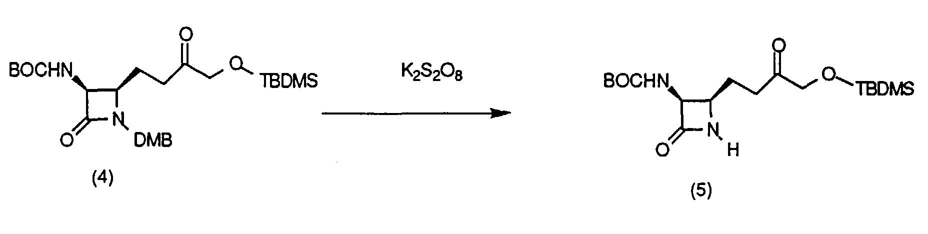

To a solution of 830 g (1.50 Mol)(2R,3S)-[2-[4-(tert-butyl-dimethyl-silanyloxy)-3-oxo-butyl]-1-(2,4-dimethoxy-benzyl)-4-oxo-azetidin-3-yl]-carbamic acid tert-butyl ester (4) in 8.3 l acetonitrile and 4.15 l water are added in intervals of 25 min three portions of 415 g (1.53 Mol) potassium persulfate at 72-75°C. The pH is kept at 4.9-5.3 by addition of a 10% solution of sodium carbonate (3.75 l). The mixture is stirred at the indicated temperature for 2 h and then cooled to room temperature. The solids are removed by filtration and washed with 5.6 l ethyl acetate. The filtrates are thoroughly mixed. The phases are separated and to the organic layer is added 2.54 l sodium bisulfite 40% in water. The phases are thoroughly mixed, cooled to 5-0°C, stirred for 1 h. The solids are removed by filtration and washed with 0.62 l ethyl acetate. The combined filtrates are stirred and the phases are allowed to separate. The organic phase is consecutively washed with aqueous solutions of 1.86 l sodium chloride (10%), 1.86 l sodium carbonate (5%) and twice with 1.86 l sodium chloride (10%), dried over 1.15 kg sodium sulfate and filtered. The solids are washed with 1.25 l ethyl acetate. To the combined filtrates is added 1.25 kg silica gel 60 ( 230-400 mesh) and the mixture is concentrated under aspirator vacuum. The remaining solid is charged onto a column containing 3.5 kg silica gel 60 (230-400 mesh). The product is eluted with a gradient of n-hexane : ethyl acetate = 2 : 1 to 1 : 2. The pure product containing fractions are combined and concentrated under aspirator vacuum and dried 0,1 torr at 45 °C to yield 464 g (2R,3S)-[2-[4-(tert-butyl-dimethyl-silanyloxy)-3-oxo-butyl]-4-oxo-azetidin-3-yl]-carbamic acid tert-butyl ester (5) as a yellow amorphous solid (80% Th.).
¹H-NMR (DMSO): 0,04 (s,6H); 0.87 (s,9H); 1.39 (s,9H); 1.58 (m,2H); 2.36 (m,2H); 3.48 (m,1H); 4.24 (s,2H); 4.73 (dd,J=10Hz,J=5Hz,1H); 7.69 (d,J=10hz,1H); 8.20 (s,1H) ppm.
MS (EI): 313 (M-t-butoxy); 273 [M-(t-butyl)₂].
IR (KBr): 1738 cm⁻¹(ν β-Lactam CO)

| | | | |
|---|---|---|---|
| M A calc.for C₁₈H₃₄N₂O₆Si): | C:55.93; | H:8.87; | N:7.25; |
| found: | C:55.73; | H:8.91; | N:7.05 (%) |

### Example 4

### Synthesis of (2R,3S)-{3-tert-Butoxycarbonylamino-2-[4-(tert-butyl]-dimethyl-silanyloxy)-3-oxo-butyl]-4-oxo-azetidin-1-yl}-oxo-acetic acid tert-butyl ester (7)

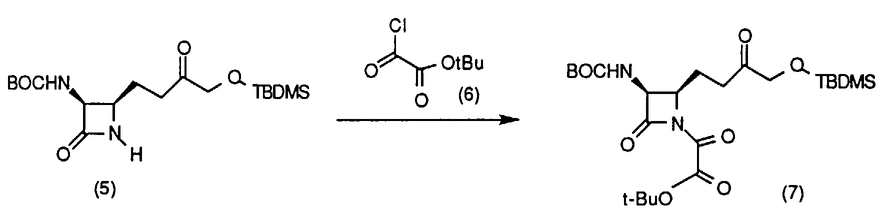

wherein t-Bu stands for tert.butyl.
To a solution of 490.7 g (3.0 Mol) chloro-oxo-acetic acid tert-butyl ester (6) in 4.41 l methylene chloride is added 577 g (5.7 Mol) calcium chloride. The suspension is cooled to 0-5°C. A cold (0°C) solution of 575 g (1.48 Mol) (2R,3S)-[2-[4-(tert-butyl-dimethyl-silanyloxy)-3-oxo-butyl]-4-oxo-azetidin-3-yl]-carbamic acid tert-butyl ester (5) and 385 g (3.0 Mol) N,N-diisopropylethylamine is added at such a rate that the temperature does not rise above 5°C (1.5 h). The reaction mixture is warmed to 23°C and stirred for 1 h. The solids are removed by filtration and washed with 1.0 l methylene chloride. The combined filtrates are extracted with 1.5 l water, twice with 1.5 l sodium bicarbonate 5% and once again with 1.5 l water. The aqueous phases are extracted with 1.0 l methylene chloride. The combined organic phases are dried over 500 g sodium sulfate and filtered. The solids are washed with 1 l methylene chloride. The combined filtrates are concentrated to a volume of 5.0 l. To this solution 20 l n-hexane is added and the mixture is concentrated to a volume of 10 l. The product is allowed to crystallize while stirring at room temperature for 16 h. The precipitate is collected by filtration, washed with 4 l n-hexane and dried at 0,1 torr at 40 °C to yield 550 g of (2R,3S)-{3-tert-butoxy carbonylamino-2-[4-(tert-butyl-dimethyl-silanyloxy)-3-oxo-butyl]-4-oxo-azetidin-1-yl}-oxo-acetic acid tert-butyl ester (7) as colourless crystals (69% Th.); m.p.: 102-103°C
¹H-NMR (DMSO): 0,09 (s,6H); 0.92 (s,9H); 1.45 (s,9H); 1.56 (s,9H); 2.01 (m,2H); 2.76 (m,2H); 4.20 (s,2H); 4.35 (m,1H); 5.27 (dd,J=9Hz,J=6Hz,1H) ppm.
MS (ISP): 532 (M+NH₄⁺); 537 (M+Na⁺).
IR (KBr): 1811 cm⁻¹(ν β-Lactam CO)

| | | | |
|---|---|---|---|
| M A (calc.for C₂₄H₄₂N₂O₈Si): | C:56.01; | H:8.23; | N:5.44; |
| found: | C:55.77; | H8.30; | N:5.43 (%) |

### Example 5

### Synthesis of (6R,7S)-7-tert-Butoxycarbonylamino-3-(tert-butyl-dimethyl-silanyloxymethyl)-8-oxo-1-aza-bicyclo[4.2.0]oct-2-ene-carboxylic acid tert-butyl ester (8)

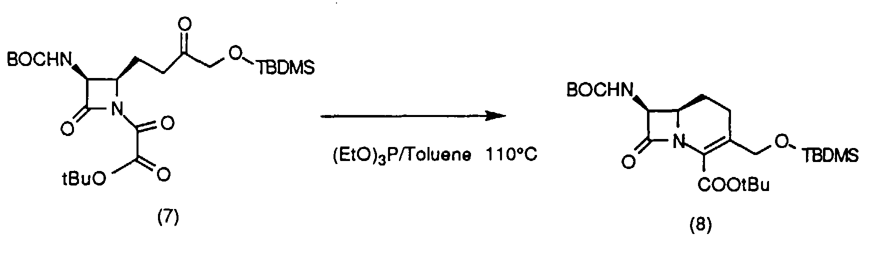

A solution of 560 g (1.09 Mol) (2R,3S)-{3-tert-butoxycarbonylamino-2-[4-(tert-butyl-dimethyl-silanyloxy)-3-oxo-butyl]-4-oxo-azetidin-1-yl}-oxo-acetic acid tert-butyl ester (7), 890 g (5.35 Mol) triethyl phosphite and 11.1 g (0.10 Mol) hydroquinone in 3.26 l toluene is heated under argon to 70-80°C for 3 h. The mixture is diluted with 2.3 l toluene and heated to reflux for 24 h. The resulting brown solution is completely evaporated at 60°C, first at aspirator then at high vacuum. The oily residue is taken up in 6.5 l n-hexane and extracted 6 times with 2.0 l of a 2 : 1 mixture of methanol and water. The aqueous phases are extracted 3 times with 2.0 l n-hexane. The combined hexane phases are dried over 1.1 kg sodium sulfate and filtered. The solids are washed with 2.0 l n-hexane and the combined filtrates are concentrated under aspirator vacuum at 40 °C whereby 415 g (6R,7S)-7-tert-butoxycarbonylamino-3-(tert-butyl-dimethyl-silanyloxymethy])-8-oxo-1-aza-bicyclo[4.2.0]oct-2-ene-carboxylic acid tert-butyl ester (8) is obtained as yellowish solid (72% Th.).
¹H-NMR (DMSO): 0,06 (s,3H); 0.07 (s,3H); 0.89 (s,9H); 1.45 (s,9H); 1.58 (s,9H); 1.58 (m,1H); 2.1 (m,1H); 2.45 (m,2H); 3.8 (m,1H); 4.40 (d,J=14Hz,1H); 4.71 (d,J=14Hz,1H); 5.03 (d,J=8Hz,1H); 5.14 (dd,J=8Hz,J=5Hz,1H) ppm.
MS (ISP): 483.3 (M+H⁺); 500.4 (M+NH₄⁺); 505.4 (M+Na⁺).
IR (KBr): 1760 cm⁻¹(ν β-Lactam CO)

| | | | |
|---|---|---|---|
| M A (calc.for C₂₄H₄₂N₂O₆Si): | C:59.72; | H:8.77; | N:5.80; |
| found: | C:59.44; | H:8.86; | N:5.68 (%) |

### Example 6

### Synthesis of (6R,7S)-7-tert-Butoxycarbonylamino-3-hydroxymethyl-8-oxo-1-aza-bicyclo[4.2.0]-oct-2-ene-2-carboxylic acid tert-butyl ester (8)

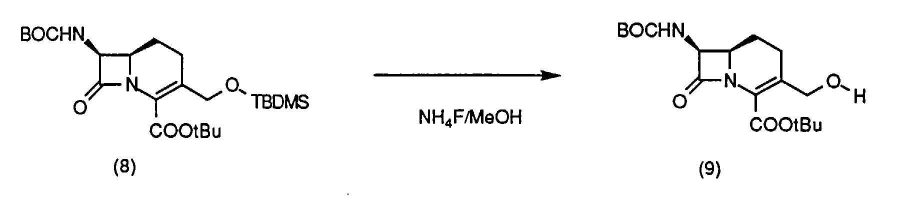

To a solution of 257 g (0.42 Mol) (6R,7S)-7-tert-butoxycarbonylamino-3-(tert-butyl-dimethyl-silanyloxymethyl)-8-oxo-1-aza-bicyclo[4.2.0]oct-2-ene-carboxylic acid tert-butyl ester (8) in 6.5 l methanol is added 128 g (3.46 Mol) ammonium fluoride. The mixture is stirred at 25 °C for 21 h. The mixture is concentrated to a volume of 0.6 l under aspirator vacuum at 40-45°C. The residue is stirred with 1.2 l water at room temperature for 1 h. The precipitate is collected by filtration, washed twice with 50 ml water, twice with 100 ml of a 1:1 mixture of methanol and water and twice with 50 ml pentane. The solid is dried at 0,1 torr at 30°C whereby 141.7 g (6R,7S)-7-tert-butoxycarbonylamino-3-hydroxymethyl-8-oxo-1-aza-bicyclo[4.2.0]-oct-2-ene-2-carboxylic acid tert-butyl ester (9) is obtained as colourless crystals (89% Th.);
m.p.: 190-191°C
MS (ISP): 369.3 (M+H⁺); 391.2 (M+Na⁺).
IR (KBr): 1762 cm⁻¹(ν β-Lactam CO)

| | | | |
|---|---|---|---|
| M A (calc.for C₁₈H₂₈N₂O₆): | C:58.68; | H:7.66; | N:7.60; |
| found: | C:58.88; | H:7.69; | N:7.60 (%) |

### Example 7

### Synthesis of (6R,7S)-7-tert-Butoxycarbonylamino-3-formyl-8-oxo-1-aza-bicyclo[4.2.0]oct-2-ene-2-carboxylic acid tert-butyl ester (10)

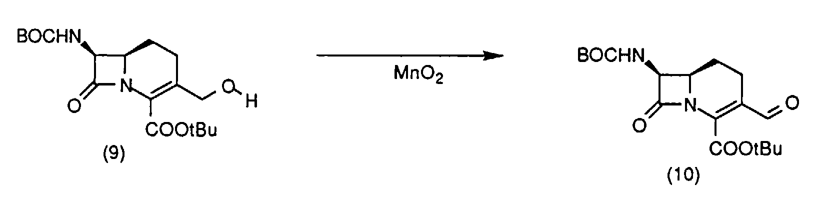

A solution of 194 g (0.52 Mol) of (6R,7S)-7-tert-butoxycarbonylamino-3-hydroxymethyl-8-oxo-1-aza-bicyclo[4.2.0]-oct-2-ene-2-carboxylic acid tert-butyl ester (9) in 4.85 l of methylene chloride is stirred with 455 g (0.52 Mol) manganese(IV) oxide at 22-25°C for 15 h. Another 45.5 g manganese(IV) oxide is added and stirring is continued for 24 h. A further 45.5 g of manganese(IV) oxide is added and stirring is continued for 3.5 h. The solids are removed by filtration over 500 g dicalite and washed with 3 l methylene chloride. The combined filtrates are concentrated under aspirator vacuum and dried at 0,1 torr at 40°C. The residue is dissolved in 182 ml methylene chloride, diluted with 730 ml of pentane and stirred at room temperature for 30 min. The resulting solid is collected by filtration, washed with 140 ml pentane and dried at 0,1 torr at 40°C to yield 158.5 g (6R,7S)-7-tert-butoxycarbonyiamino-3-formyl-8-oxo-1-aza-bicycio[4.2.0]oct-2-ene-2-carboxylic acid tert-butyl ester (10) as colourless crystals (83% Th.); m.p.: 194-195°C
¹H-NMR (CDCl₃): 1.40 (m,1H); 1.45 (s,9H); 1.58 (s,9H); 2.15 (m,2H); 2.9 (m,1H); 3.90 (m,1H); 5.00 (d,J=8Hz,1H); 5.25 (dd,J=8Hz,J=5Hz,1H); 9.94 (s,1H) ppm.
MS (ISP): 367.3 (M+H⁺); 384.3 (M+NH₄⁺); 389.3 (M+Na⁺).
IR (KBr): 1786 cm⁻¹(ν β-Lactam CO)

| | | | |
|---|---|---|---|
| M A (calc.for C₁₈H₂₆N₂O₆): | C:59.00; | H:7.18; | N:7.65; |
| found: | C:58.84; | H:7.17; | N:7.53 (%) |

### Example 8

### 8.1. Synthesis of (E)-(6R,7S)-7-tert-Butoxycarbonylamino-8-oxo-3-[2-oxo-1-(2,2,2-trifluoro-ethyl)-pyrrolidin-3-ylidenementhyl]-1-azabicyclo[4.2.0]oct-2-ene-2-carboxylic acid tert-butyl ester

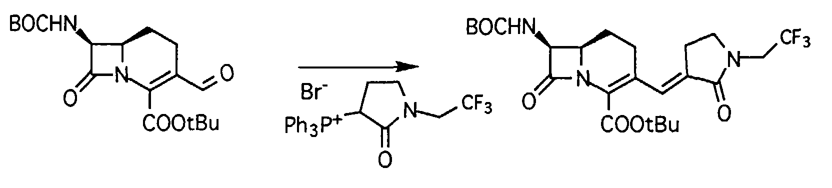

A mixture of 0.50 g (6R,7S)-7-tert-butoxycarbonylamino-3-formyl-8-oxo-1-aza-bicyclo[4.2.0]oct-2-ene-2-carboxylic acid tert-butyl ester (10) and 0.96 g rac-[2-oxo-1-(2,2,2-trifluoroethyl)-3-pyrrolidinyl]-triphenylphosphonium bromide in 6.0 ml 1,2 epoxybutane is heated to reflux for 6 h. The solvent is evaporated and the residue is purified by chromatography on silica gel using n-hexane : ethyl acetate = 3 : 1 as eluent. The title compound, (E)-(6R,7S)-7-tert-butoxycarbonylamino-8-oxo-3-[2-oxo-1-(2,2,2-trifluoro-ethyl)-pyrrolidin-3- ylidenemethyl]-1-azabicyclo[4.2.0]oct-2-ene-2-carboxylic acid tert-butyl ester, is obtained as white foam (0.55 g;78% Th).
¹H-NMR (CDCl₃): inter alia 1.45 (s,9H); 1.57 (s,9H); 2.00-2.20 (m,1H); 2.35-2.55 (m,1H); 2.75-3.10 (m,3 H); 3.50-3.65 (m,2H); 3.77-3.87 (m,1H); 3.90-4.10 (m,2H); 5.05-5.25 (m,2H); 7.60 (s,1H) ppm.
MS (ISP): 516 (M+H⁺); 533 (M+NH₄⁺); 538 (M+Na⁺).
IR (KBr): 1768 cm⁻¹(ν β-Lactam CO)
In similar manner the following compounds were obtained from (6R,7S)-7-tert-butoxycarbonylamino-3-formyl-8-oxo-1-aza-bicyclo[4.2.0]oct-2-ene-2-carboxylic acid tert-butyl ester (10) by Wittig reaction with the corresponding phosphonium bromides.

### 8.2. (E)-(6R,7S-tert-Butoxycarbonylamino-3-[1-(4-tert-butoxycarbonyloxy-phenyl)-2-oxo-pyrrolidin-3-ylidenemethyl]-8-oxo-1-aza-bicyclo[4.2.0]oct-2-ene-2-carboxylic acid tert-butyl ester as yellowish crystals, m.p.: 213°C (dec.)

¹H-NMR (DMSO): inter alia 1.40 (m,9H); 1.49 (s,9H); 1.51(s,9H);1.6-2.0(m,2H); 3.75-3.95 (m,3H); 5.20 (dd,J=8Hz,J=5Hz,1H); 7.22 (d,J=9Hz,2H); 7.45 (s,1H); 7.76 (d,J=8Hz,1H); 7.82 (d,J=9Hz,2H) ppm
MS (ISP): 626.5 (M+H⁺); 643.5 (M+NH₄⁺).
IR (KBr): 1756 cm⁻¹(ν β-Lactam CO)

| | | | |
|---|---|---|---|
| M A (calc.for C₃₃H₄₃N₃O₉): | C:63.35; | H:6.93; | N:6.72; |
| found : | C:62.92; | H:7.71; | N:6.60 (%) |

### 8.3. (E)-(6R,7S)-tert-Butoxycarbonylamino-3-[1-(3-nitro-phenyl)-2-oxo-pyrrolidn-3-ylidenemethyl]-8-oxo-1-aza-bicyclo[4.2.0]oct-2-ene-2-carboxylic acid tert-butyl ester as yellow solid

¹H-NMR (CDCl₃): inter alia 1.49 (s,9H); 1.58 (s,9H); 1.95-2.15 (m,1H); 2.35-2.55 (m,1H); 3.00-3.50 (m,3 H); 3.70-4.00 (m,3H); 3.9-4.05 (m,1H); 5.24 (dd,J=8Hz,J=5Hz,1H); 6.13 (d,J=8Hz,1H); 7.27-7.38 (m,1H); 7.70-7.80 (m,1H); 7.85-7.93 (m,1H); 7.95-8.05 (m,1H); 8.40 (s,1H) ppm.
MS (ISP): 555.4 (M+H⁺); 572.5 (M+NH₄⁺); 577.4 (M+Na⁺).
IR (KBr): 1768 cm⁻¹(ν β-Lactam CO)

### 8.4. (E)-(6R,7S)-7-tert-Butoxycarbonylamino-8-oxo-3-(2-oxo-1-pyridin-2-yl-pyrrolidin-3-ylidenemethyl)-1-aza-bicyclo[4.2.0]oct-2-ene-2-carboxylic acid tert-butyl ester

m.p.: 236°C (dec.)
¹H-NMR (CDCl₃): 1.46 (m,9H); 1.58 (s,9H); 2.37-2.58 (m,1H); 2.93-3.32 (m,3H); 3.75-3.85 (m,1 H); 3.90-4.11 (m,2H); 5.25 (dd,J=8Hz,J=5Hz,1H); 5.89 (d,J=8Hz,2H); 6.82-6.92 (m,1H); 7.50-7.61 (m,1H); 7.87 (s,1H); 8.10-8.18 (m,1H); 8.48 (d,J=8.5Hz,1H) ppm.
MS (ISP): 511.4 (M+H⁺); 533.4 (M+Na⁺).
IR (KBr): 1767 cm⁻¹(ν β-Lactam CO)

### 8.5. (E)-(6R,7S)-7-tert-Butoxycarbonylamino-8-oxo-3-(2-oxo-1-pyridin-3-yl-pyrrolidin-3-ylidenemethyl)-1-aza-bicyclo[4.2.0]oct-2-ene-2-carboxylid acid tert-butyl ester as beige crystals, m.p.: 243°C (dec.)

¹H-NMR (CDCl₃): inter alia 1.46 (s,9H); 1.57 (s,9H); 2.32-2.52 (m,1H); 2.96-3.17 (m,2H); 3.27-3.50 (m,1H); 3.59-3.95 (m,3H); 5.24 (dd,J=8Hz,J=5Hz,1H); 6.26 (d,J=8Hz,1H); 7.00-7.10 (m,1H); 7.86 (s,1H); 8.00-8.10 (m,1H); 8.17-8.25 (m,1H); 8.70-8.80 (m,1H) ppm.
MS (ISP): 511.2 (M+H⁺).
IR (KBr): 1769 cm⁻¹(ν β-Lactam CO)

| | | | |
|---|---|---|---|
| M A (calc.for C₂₇H₃₄N₄O₆): | C:63.51; | H:6.71; | N:10.97; |
| found: | C:62.98; | H:6.72; | N:10.97 (%) |

### 8.6. (E)-(6R,7S)-7-tert-Butoxycarbonylamino-8-oxo-3-(2-oxo-1-pyridin-3-yl-pyrrolidin-3-ylidenemethyl)-1-aza-bicyclo[4.3.0]oct-2-ene-2-carboxylic acid tert-butyl ester as white crystals, m.p.: 242°C (dec.)

¹H-NMR (CDCl₃): inter alia 1.46 (s,9H); 1.58 (s,9H); 1.95-2.10 (m,1H); 2.30-2.50 (m,1H); 2.90-3.15 (m,2 H); 3.20-3.40 (m,1H); 3.55-3.85 (m,3H); 5.25 (dd,J=8Hz,J=5Hz,1H); 6.14 (d,J=8Hz,1H); 7.48 (d,J=6Hz,2H); 7.88 (s,1H); 8.34 (d,J=6Hz,2H) ppm.
MS (ISP): 511.5 (M+H⁺).
IR (KBr): 1771 cm⁻¹(ν β-Lactam CO)

| | | | |
|---|---|---|---|
| M A (calc.for C₂₇H₃₄N₄O₆+ 0.5563 mol C₄H₈O₂): | C:62.06; | H:7.92; | N:9.91 |
| found: | C:61.97; | H:7.79; | N:9.97 |

### 8.7. (E)-(6R,7S)-7-tert-Butoxycarbonylamino-8-oxo-3-(2-oxo-1-pyridin-3-yl-pyrrolidin-3-ylidenemethyl)-1-aza-bicyclo[4.2.0]oct-2-ene-2-carboxylic acid tert-butyl ester as yellow solid.

| | | | |
|---|---|---|---|
| M A (calc.for C₂₇H₄₀N₄O₆+0.5563 Mol C₄H₈O₂) | C:62.06; | H:7.92; | N:9.91 |
| found: | C:61.97; | H:7.79; | N:10.08 |

### Example 9

### (Alkylation of R² = pyridin-2-yl)

### 9.1. Synthesis of (E)-(6R,7S)-3-[3-(2-tert-Butoxycarbonyl-7-tert-butoxycarbonylamino-8-oxo-1-aza-bicyclo[4.2.0]oct-2-en-3-ylmethylene)-2-oxo-pyrrolidin-1-yl]-1-methyl-pyridinium iodide

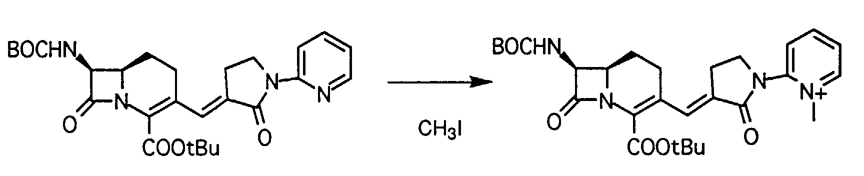

To a solution of 9.50 g (E)-(6R,7S)-7-tert-butoxycarbonylamino-8-oxo-3-(2-oxo-1-pyridin-3-yl-pyrrolidin-3-ylidenemethyl)-1-aza-bicyclo[4.2.0]oct-2-ene-2-carboxylic acid tert-butylester in 100 ml N,N-dimethylformamide are added 3.17 g methyl iodide and the mixture is stirred at room temperature for 64 h. The solvent is removed by evaporation at 0,1 torr. The residue is triturated in 100 ml ethyl acetate. The yellow solid is collected by filtration. The title compound is obtained as N,N-dimethylformamide solvate (12g, 90% Th).
m.p.: 190°C (dec.)
¹H-NMR (CDCl₃): inter alia 1.46 (s,9H); 1.56 (s,9H); 1.90-2.15 (m,1H); 2.35-2.55 (m,1H); 2.90-3.50 (m,3 H); 3.70-3.85 (m,1H); 3.9-4.05 (m,1H);4.16-4.35 (m,1H); 4.69 (s,3H); 5.28 (dd,J=8Hz,J=5Hz,1H); 6.21 (d,J=8Hz,1H); 7.88 (s,1H); 8.07 (dd,J=15Hz,J=9Hz,1H); 8.79 (d,J=9Hz,1H); 9.09 (d,J=15Hz,1H); 9.62 (s,1H) ppm.
MS (ISP): 525.5 (M⁺)
IR (KBr): 1765 cm⁻¹(ν β-Lactam CO)

| | | | |
|---|---|---|---|
| MA (calc.for C₂₈H₃₇N₄O₆I + 0.8 mol C₃H₇NO: | C:51.35; | H:6.04; | N:9.46; |
| found: | C:51.11; | H:5.83; | N:9.64 |

In an analogous manner is obtained

### 9.2. (E)-(6R,7S)-4-[3-(2-tert-Butoxycarbonyl-7-tert-butoxycarbonylamino-8-oxo-1-aza-bicyclo[4.2.0]oct-2-en-3-ylmethylene)-2-oxo-pyrrolidin-1-yl]-1-methyl-pyridinium iodide.

m.p.: 163°C (dec.)
¹H-NMR (DMSO): inter alia 1.40 (s,9H); 1.52 (s,9H); 1.50-2.00 (m,2H); 2.4-2.90 (m,2H); 3.1-3.40 (m,2 H); 3.80-4.10 (m,3H); 4.20 (s,3H); 5.25 (dd,J=8Hz,J=5Hz,1H); 7.57 (s,1H); 7.75 (d,J=8Hz,1H); 8.33 (d,J=7Hz,2H); 8.78 (d,J=7Hz,1H) ppm.
MS (ISP): 525.7 (M⁺)
IR (KBr): 1768 cm⁻¹(ν β-Lactam CO)

### 9.3. (E)-(6R,7S)-2-[3-(2-tert-Butoxycarbonyl-7-tert-butoxycarbonylamino-8-oxo-1-aza-bicyclo[4.2.0]oct-2-en-3-ylmethylene)-2-oxo-pyrrolidin-1-yl]-1-methyl-pyridinium tetrafluoroborate.

m.p.: 179°C (dec.)
¹H-NMR (DMSO): inter alia 1.41 (s,9H); 1.49 (s,9H); 1.50-2.00 (m,2H); 2.5-3.0 (m,2H); 3.1-3.50 (m,2 H); 3.80-4.10 (m,3H); 4.16 (s,3H); 5.24 (dd,J=8Hz,J=5Hz,1H); 7.51 (s,1H); 7.77 (d,J=8Hz,1H); 8.00-8.10 (m,1H); 8.16-8.25 (d,J=8Hz,1H); 8.63-8.75 (m,1H); 9.03-9.13 (m,1H) ppm.
MS (ISP): 525.5 (M⁺)
IR (KBr): 1770 cm⁻¹(ν β-Lactam CO)

### 9.4. (E)-(6R,7S)-4-[3-(2-tert-Butoxycarbonyl-7-tert-butoxycarbonylamino-8-oxo-1-aza-bicydo[4.2.0]oct-2-en-3-ylmethylene)-2-oxo-pyrrolidin-1-yl]-1-[(4-tert-butoxycarbonyloxy-3-fluoro-phenylcarbomoyl)-methyl]-pyridinium bromide

m.p.: 176°C
¹H-NMR (DMSO): inter alia 1.40 (s,9H); 1.48 (s,9H); 1.52 (s,9H); 1.50-2.00 (m,2H); 2.4-2.90 (m,2H); 3.1-3.40 (m,2 H); 3.80-4.10 (m,3H); 5.25 (dd,J=8Hz,J=5Hz,1H); 5.50 (s,2H); 7.37 (m, 2H); 7.59 (s,1H); 7.74 (m,2H); 8.41 (d,J=7Hz,2H); 8.84 (d,J=7Hz,1H); 10.94 (s,1H) ppm.
MS (ISP): 778.4 (M⁺)
IR (KBr): 1768 cm⁻¹(ν β-Lactam CO)

### Example 10

### 10.1. Synthesis of (E)-(6R,7S)-7-Amino-8-oxo-3-[2-oxo-1-(2,2,2-trifluoro-ethyl)-pyrrolidin-3-ylidenemethyl]-1-aza-bicyclo[4.2.0]oct-2-ene-2-carboxylic acid

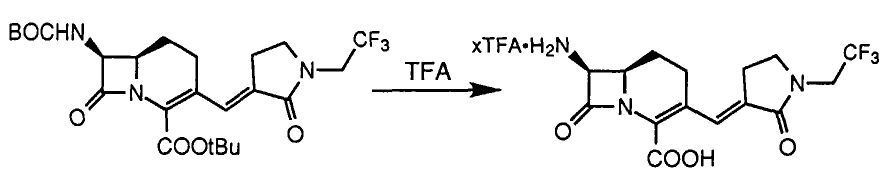

wherein TFA stands for trifluoroacetate and x is the molar fraction as determined by elemental analysis.
A solution of 1.28 g (E)-(6R,7S)-7-tert-butoxycarbonylamino-8-oxo-3-[2-oxo-1-(2,2,2-trifluoro-ethyl)-pyrrolidin-3-ylidenemethyl]-1-azabicyclo[4.2.0]oct-2-ene-2-carboxylic acid tert-butyl ester in 15 ml trifluoroacetic acid is stirred at room temperature for 2 h. The solvent is removed by evaporation. The residue is triturated with ethyl acetate. The solid is collected by filtration. The title compound is obtained as beige powder. (0.82g, 86 % Th);
m.p.: 237 °C (dec)
¹H-NMR (DMSO): inter alia 1.54-1.80 (m,1H); 1.85-2.00 (m,1H); 2.75-3.25 (m,3H); 3.4-3.60 (m,2 H); 3.67-3.82 (m,1H); 4.16 (qu,J=10Hz,2H); 4.50 (d,J=5Hz,1H); 7.39 (s,1H)ppm.
MS (ISP): 360.4 (M+H⁺); 377.4 (M+NH₄⁺); 382.2 (M+Na⁺).
IR (KBr): 1769 cm⁻¹(ν β-Lactam CO)

| | | | |
|---|---|---|---|
| M A (calc.for C₁₅H₁₆N₃O₄F₃ + 0.228 mol C₂H₁O₂F₃: | C:48.18; | H:4.25; | N:10.90; |
| found: | C:48.13; | H:4.28; | N:10.96 |

In a similar manner the following 7-amino-carbacephemcarboxylic acids are obtaind from their corresponding protected precursors.

### 10.2. (E)-(6R,7S)-7-Amino-3-[1-(4-hydroxy-phenyl)-2-oxo-pyrrolidin-3-ylidenemethyl]-8-8-oxo-1-aza-bicyclo[4.2.0]oct-2-ene-2-carboxylic acid as crystallin solid from trifluoroacetic acid

m.p.: 151-153 °C (dec)
¹H-NMR (DMSO): inter alia 1.60-2.00 (m,2H); 2.80-3.30 (m,3H); 3.75-4.05 (m,3H); 4.92 (d,J=5Hz,1H); 6.78 (d,J=9Hz,2H); 7.47 (s,1H); 7.56 (d,J=9Hz,2H) ppm.
MS (ISN): 368.2 (M-H⁺).
IR (KBr): 1769 cm⁻¹(ν β-Lactam CO)

| | | | |
|---|---|---|---|
| M A calc.for C₁₉H₁₉N₃O₅ + 1.8 mol C₂H₁O₂F₃: | C:47.25; | H:3.65; | N:7.31; |
| found: | C:47.25; | H:3.80; | N:7.30 (%) |

### 10.3 (E)-(6R,7S)-7-Amino-3-[1-(3-nitro-phenyl)-2-oxo-pyrrolidin-3-ylidenemethyl]-8-oxo-1-aza-bicyclo[4.2.0]oct-2-ene-2-carboxylic acid as yellow crystals from ethyl acetate, m.p.: 242-243°C (dec.)

¹H-NMR (DMSO): inter alia 1.60-2.15 (m,2H); 3.80-4.05 (m,3H); 4.50 (d,J=5Hz,1H); 7.55 (s,1H); 7.65-7.75 (m,1H); 8.00-8.15 (m.2H); 8.86(s br,1H)ppm.
MS (ISN): 397.2 (M-H⁺).
IR (KBr): 1779 cm⁻¹(ν β-Lactam CO)

| | | | |
|---|---|---|---|
| M A (calc.for C₁₉H₁₈N₄O₆ + 0.322 mol C₂H₁O₂F₃ and 1.17% water): | C:54.23; | H:4.25; | N:12.88; |
| found: | C:54.14; | H:4.54; | N:12.69 (%) |

### 10.4. (E)-(6R,7S)-7-Amino-8-oxo-3-(2-oxo-1-piperidin-1-yl-pyrrolidin-3-ylidenemethyl)-1-aza-bicyclo[4.2.0]oct-2-ene-2-carboxylic acid as yellowish solid

¹H-NMR (DMSO): inter alia 3.60-3.75 (m,1H); 4.50 (d,J=5Hz,1H); 7.25 (s,1H) ppm.
MS (ISP): 361.3 (M+H⁺); 383.4 (M+Na⁺).
IR (KBr): 1756 cm⁻¹(ν β-Lactam CO)

### 10.5. (E)-(6R,7S)-7-Amino-8-oxo-3-(2-oxo-1-piperidin-2-yl-pyrrolidin-3-ylidenemethyl)-1-aza-bicyclo[4.2.0]oct-2-ene-2-carboxylic acid as beige crystals (from water at pH 7.00)

m.p.: 272°C (dec.)
MS (ISN): 353.3 (M-H⁻).
IR (KBr): 1763 cm⁻¹(ν β-Lactam CO)

| | | | |
|---|---|---|---|
| MA (calc.for C₁₈H₁₈N₄O₄ + 0.124 mol C₂H₁O₂F₃ and 1.37% water): | C:59.48; | H:4.96; | N:15.20; |
| found: | C:59.48; | H:4.90; | N:15.14 (%) |

### 10.6. (E)-(6R,7S)-7-Amino-8-oxo-3-(2-oxo-1-piperidin-3-yl-pyrrolidin-3-ylidenemethyl)-1-aza-bicyclo[4.2.0]oct-2-ene-2-carboxylic acid trifluoroacetate (1:2) as white crystals

¹H-NMR (DMSO): inter alia 4.92 (d,J=5Hz,1H); 7.45-7.55 (m,2H); 8.25-8.35 (m.1H); 8.40-8.46 (m,1H); 9.02-9.10 (m,1H) ppm.
MS (ISP): 355.4 (M+H⁺); 377.3 (M+Na⁺).
IR (KBr): 1774 cm⁻¹(ν β-Lactam CO)

| | | | |
|---|---|---|---|
| MA (calc.for C₁₉H₁₈N₄O₆ + 2 mol C₂H₁O₂F₃): | C:45.37; | H:3.46; | N:9.62; |
| found: | C:45.28; | H:3.89; | N:9.60 (%) |

### 10.7. (E)-(6R,7S)-7-Amino-8-oxo-3-(2-oxo-1-piperidin-4-yl-pyrrolidin-3-ylidenemethyl)-1-aza-bicyclo[4.2.0]oct-2-ene-2-carboxylic acid as white crystals (from water at pH 7.00)

m.p.: 262°C (dec.)
¹H-NMR (D₂O): inter alia 1.80-2.00 (m,1H); 2.30-2.45 (m,1H); 2.6-2.85 (m,1H); 3.00-3.40 (m,3H); 4.05-4.25 (m,3H); 5.00 (d,J=5Hz,1H); 7.70 (s,1H); 8.32 (d,J=7Hz,2H); 8.63 (d,J=7Hz,2H) ppm.
MS (ISP): 355.3 (M+H⁺); 377.3 (M+Na⁺).
IR (KBr): 1760 cm⁻¹(ν β-Lactam CO)

### 10.8. (E)-(6R,7S)-7-Amino-8-oxo-3-[1-(1-methyl-pyridin-1-ium-2-yl)-2-oxo-pyrrolidin-3-ylidenemethyl]-8-oxo-1-aza-bicyclo[4.2.0]oct-2-ene-2-carboxylate tetrafluoro-borate (1:1) as yellow crystals, m.p.:183°C (dec)

¹H-NMR (DMSO): inter alia 4.16 (s,3H); 4.82 (d,J=5Hz,1H); 7.57 (s ,1H); 8.06 (t,J=8Hz,1H); 8.21 (d,J=8Hz,1H); 8.68 (t,J=8Hz,1H); 9.08 (d,J=8Hz,1H) ppm.
MS (ISP): 369.3 (M+H⁺); 391.4 (M+Na⁺).
IR (KBr): 1771 cm⁻¹(ν β-Lactam CO)

| | | | |
|---|---|---|---|
| MA (calc.for C₁₉H₂₁N₄O₄BF₄+ 0.68 mol C₂H₁O₂F₃): | C:45.82; | H:4.10; | N:10.50; |
| found: | C:45.92; | H:4.28; | N:10.21 (%) |

### 10.9. (E)-(6R,7S)-7-Amino-3-[1-(1-methyl-pyridin-1-ium-3-yl)-2-oxo-pyrrolidin-3-ylidenemethyl]-8-oxo-1-aza-bicyclo[4.2.0]oct-2-ene-2-carboxylate trifluoroacetate (1:1) as yellow crystals, m.p.:>200°C (dec)

¹H-NMR (DMSO): inter alia 4.40 (s,3H); 4.81 (d,J=5Hz,1H); 7.62 (s ,1H); 8.15 (dd,J=9Hz,J=6Hz,1H); 8.75 (d,J=6Hz,1H); 8.87 (d,J=9Hz,1H); 9.48 (s,1H) ppm

### 10.10. (E)-(6R,7S)-7-Amino-3-[1-(1-methyl-pyridin-1-ium-4-yl)-2-oxo-pyrrolidin-3-ylidenemethyl]-8-oxo-1-aza-bicyclo[4.2.0]oct-2-ene-2-carboxylate-hydroiodide (1:1) as yellow crystals, m.p.:>200°C (dec)

¹H-NMR (DMSO): inter alia 4.20 (s,3H); 4.94 (d,J=5Hz,1H); 7.66 (s,1H); 8.32 (d,J=7Hz,2H); 8.81 (d,J=7Hz,2H) ppm
IR (KBr): 1774 cm⁻¹(ν β-Lactam CO)
MS (ISP): 369.4 (M+H⁺); 391.4 (M+Na⁺).

### 10.11. (E)-(6R,7S)-7-Amino-3-[1-[1-[(3-fluoro-4-hydroxy-phenylcarbamoyl)-methyl]-pyridin-1-ium-4-yl]-2-oxo-pyrrolidin-3-ylidenemethy]-8-oxo-1-aza-bicyclo[4.2.0]oct-2-ene-2-carboxylate m.p.: 191°C (dec)

¹H-NMR (DMSO): inter alia 4.00 (m,3H); 4.92 (d,J=5Hz,1H); 5.44 (s,2H); 6.92 (t,1H); 7.10 (d,1H); 7.50 (d,1H); 7.67 (s ,1H); 8.39 (d,J=7Hz,2H); 8.81 (d,J=7Hz,2H); 9.76 (s,1H); 10.60 (s,1H) ppm
IR (KBr): 1771 cm⁻¹(ν β-Lactam CO)
MS (ISP): 522.4 (M+H⁺).

### Example 11

### 11.1. Synthesis of (6R,7S)-7-[(Z)-2-(2-Amino-thiazol-4-yl)-2-cyclopentyloxy-imino-acetylamino]-8-oxo-3-[(E)-2-oxo-1-(2,2,2-trifluoro-ethyl)-pyrrolidin-3-ylidenemethyl]-1-aza-bicyclo[4.2.0]oct-2-ene-2-carboxylic acid sodium salt (1:1)

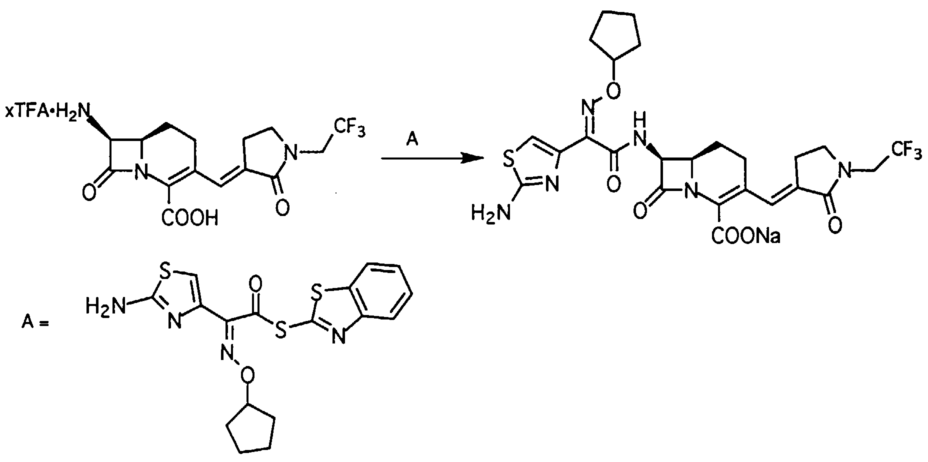

A solution of 0.15 g (E)-(6R,7S)-7-amino-8-oxo-3-[2-oxo-1-(2,2,2-trifluoro-ethyl)-pyrrolidin-3-ylidenemethyl]-1-aza-bicyclo[4.2.0]oct-2-ene-2-carboxylic acid and 0.25 g (2-amino-thiazol-4-yl)-cyclopentyloxyimino-thioacetic acid S-benzothiazol-2-yl ester in 1.00 ml dimethylformamid is stirred at room temperature for 16 h. The reaction mixture is distributed between water and ethyl acetate at pH 7.00. (1N NaOH is used to adjust the pH) The aqueous phase is purified by chromatography on MCI-gel CHP2OP (75-150 µ) with a continuous gradient of water to 30% acetonitrile in water.The product fractions are combined, concentrated to a volume of ca 25 ml and lyophilised. The title compound is obtained as a slightly beige lyophilisate. yield 0.21 g ( 81% Th)
¹H-NMR (DMSO): inter alia 1.40-1.90 (m,10H); 2.86-3.20 (m,2H); 3.66-3.80 (m,1H); 4.00-4.20 (m,2H); 4.60-4.70 (m,1H); 5.30 (dd, J=8Hz,J=5Hz,1H); 6.70 (s,1H); 7.22 (s,2H); 7.48 (s,1H); 9.22 (d,J=8Hz,1H)ppm.
MS (ISP): 597.3 (M-Na⁺ +2H⁺).
IR (KBr): 1748 cm⁻¹(ν β-Lactam CO)

| | | | |
|---|---|---|---|
| MA (calc.for C₂₅H₂₆N₆O₆F₃SNa + 6.88%water: | C:48.54; | H:4.24; | N:13.59; |
| found: | C:48.24; | H:4.21; | N:13.58 (%) |

In an analogous manner the following compounds were prepared:

### 11.2. (6R,7S)-7-[(Z)-2-(Amino-thiazol-4-yl)-2-cyclopentyloxyimino-acetyl-amino]-3-[(E)-1-(4-hydroxy-phenyl)-2-oxo-pyrrolidin-3-ylidenemethyl]-8-oxo-1-aza-bicyclo[4.2.0]oct-2-ene-2-carboxylic acid sodium salt (1:1)

¹H-NMR (DMSO): inter alia 1.40-1.95 (m,10H); 2.90-3.20 (m,2H); 3.64-3.83 (m,3H); 4.58-4.72 (m,1H); 5.30 (dd, J=8Hz,J=5Hz,1H); 6.71 (s,1H); 6.78 (d,J=9Hz,2H); 7.22 (s,2H); 7.51 (s,1H); 7.55 (d,J=9Hz,2H); 9.24 (d,J=8Hz,1H); 9.56 (s,1H)ppm.
MS (ISP): 607.4 (M-Na⁺ +2H⁺); 629.4 (M+H⁺).
IR (KBr): 1748 cm⁻¹(ν β-Lactam CO)

### 11.3. (6R,7S)-7-[(Z)-2-(2-Amino-thiazol-4-yl)-2-cyclopentyloxyimino-acetylamino]-3-[(E)-1-(3-nitro-phenyl)-2-oxo-pyrrolidin-3-ylidenemethyl]-8-oxo-1-aza-bicyclo[4.2.0]oct-2-ene-2-carboxylic acid sodium salt (1:1)

¹H-NMR (DMSO): inter alia 1.40-1.90 (m,10H); 3.00-3.25 (m,2H); 3.68-3.98 (m,3H); 4.60-4.73 (m,1H); 5.31 (dd, J=8Hz,J=5Hz,1H); 6.71 (s,1H); 7.22 (s,2H); 7.60-7.75 (m,2H); 7.92-8.02 (m,1H); 8.06-8.17 (m,1H); 8.77-8.87 (m,1H); 9.25 (d,J=8Hz,1H)ppm.
MS (ISP): 636.4 (M-Na⁺ +2H⁺); 658.4 (M+H⁺).
IR (KBr): 1750 cm⁻¹(ν β-Lactam CO)

### 11.4. (6R,7S)-7-[(Z)-2-(5-Amino-[1,2,4]thiadiazol-3-yl)-2-cyclopentyloximino-acetylamino]-3-[(E)-1-(4-hydroxy-phenyl)-2-oxo-pyrrolidin-3-ylidenemethyl]-8-oxo-1-aza-bicycol[4.2.0]oct-2-ene-2-carboxylic acid Na salt (1:1)

¹H-NMR (DMSO): inter alia 1.40-1.90 (m,10H); 2.90-3.25 (m,2H); 3.65-3.85 (m,3H); 4.67-4.81 (m,1H); 5.27 (dd, J=8Hz,J=5Hz,1H); 6.77 (d,J=9Hz,2H); 7.51 (s,1H); 7.54 (d,J=9Hz,2H); 8.17 (s,2H); 9.28 (d,J=8Hz,1H); 9.65 (s(br),1H)ppm.
MS (ISP): 608.5 (M-Na⁺ +2H⁺); 630.4 (M+H⁺).
IR (KBr): 1746 cm⁻¹(ν β-Lactam CO)

### 11.5. (6R,7S)-7-[(Z)-2-(2-Amino-[1,2,4]thiadiazol-3-yl)-2-cyclopentyloxyimino-acetylamino]-3-[(E)-[1-(3-nitro-phenyl)-2-oxo-pyrrolidin-3-ylidenemethyl]-8-oxo-1-aza-bicyclo[4.2.0]oct-2-ene-2-carboxylic acid Na salt (1:1)

¹H-NMR (DMSO): inter alia 1.40-1.90 (m,10H); 3.00-3.25 (m,2H); 3.67-3.96 (m,3H); 4.68-4.78 (m,1H); 5.28 (dd, J=8Hz,J=5Hz,1H); 7.61-7.83 (m,2H); 7.92-8.00 (m,1H); 8.07-8.20 (m,3H); 8.75-8.85 (m,1H); 9.28 (d,J=8Hz,1H)ppm.
MS (ISN): 635.3 (M-Na⁺); 652.3 (M-Na⁺+NH₃)
IR (KBr): 1748 cm⁻¹(ν β-Lactam CO)

### 11.6. (6R,7S)-7-[(Z)-2-(2-Amino-thiazol-4-yl)-2-cyclopentyloxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate

¹H-NMR (DMSO): inter alia 1.40-1.90 (m,10H); 3.70-3.83 (m,1H);3.90-4.08 (m,2H); 4.15 (s,3H); 4.60-4.71 (m,1H); 5.32 (dd, J=8Hz,J=5Hz,1H); 6.71 (s,1H); 7.23 (s,2H); 7.66 (s,1H); 7.94-8.04 (m,1H); 8.13-8.23 (m,1H); 8.58-8.69 (m,1H); 9.02-9.10 (m,1H); 9.27 (d,J=8Hz,1H)ppm.
MS (ISP): 304.1 (M+2H⁺)/2; 606.4 (M+H⁺).
IR (KBr): 1757 cm⁻¹(ν β-Lactam CO)

| | | | |
|---|---|---|---|
| MA (calc.for C₂₉H₃₁N₇O₆S + 9.06% water: | C:57.51; | H:5.16; | N:16.19; |
| found: | C:57.99; | H:4.76; | N:16.30 (%) |

### 11.7. (6R,7S)-7-[(Z)-2-(2-Amino-thiazol-4-yl)-2-cyclopentyloxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate

¹H-NMR (DMSO): inter alia 1.40-1.90 (m,10H); 3.65-3.96 (m,3H); 4.39 (s,3H); 4.60-4.73 (m,1H); 5.33 (dd, J=8Hz,J=5Hz,1H); 6.72 (s,1H); 7.23 (s,2H); 7.66 (s,1H); 8.04-8.18 (m,1H); 8.66-8.75 (m,1H); 8.86-8.96 (m,1H); 9.26 (d,J=8Hz,1H); 9.40 (s,1H) ppm.
MS (ISP): 304.0 (M+2H⁺)/2; 606.4 (M+H⁺).
IR (KBr): 1757 cm⁻¹(ν β-Lactam CO)

| | | | |
|---|---|---|---|
| MA (calc.for C₂₉H₃₁N₇O₆S + 9.06% water: | C:57.51; | H:5.16; | N:16.19; |
| found: | C:57.39; | H:5.20; | N:16.04 (%) |

### 11.8. (6R,7S)-7-[(Z)-2-(2-Amino-[1,2,4]thiadiazol-3-yl)-cyclopentyloxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate

¹H-NMR (DMSO): inter alia 1.40-1.90 (m,10H); 3.66-3.98 (m,3H); 4.38 (s,3H); 4.68-4.80 (m,1H); 5.30 (dd, J=8Hz,J=5Hz,1H); 7.68 (s,1H), 8.04-8.21 (m,3H); 8.63-8.72 (m,1H); 8.80-8.90 (m,1H); 9.29 (d,J=8Hz,1H); 9.40 (s,1H) ppm.
MS (ISP): 607.4 (M+H⁺).
IR (KBr): 1756 cm⁻¹(ν β-Lactam CO)

### 11.9. (6R,7S)-7-[(Z)-2-(2-Amino-thiazol-4-yl)-2-cyclopentyloxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate

¹H-NMR (DMSO): inter alia 1.40-1.90 (m,10H); 3.71-4.00 (m,3H); 4.17 (s,3H); 4.60-4.71 (m,1H); 5.33 (dd, J=8Hz,J=5Hz,1H); 6.71 (s,1H); 7.23 (s,2H); 7.70 (s,1H); 8.27 (d,J=6Hz,2H); 8.74 (d,J=6Hz,1H); 9.25 (d,J=8Hz,1H) ppm.
MS (ISP): 606.5 (M+H⁺).
IR (KBr): 1756 cm⁻¹(ν β-Lactam CO)

### Example 12

### Synthesis of (6R,7S)-7-[(Z)-2-(2-Amino-thiazol-4-yl)-2-hydroxyimino-acetylamino]-8-oxo-3-[(E)-2-oxo-1-(2,2,2-trifluoro-ethyl)-pyrrolidin-3-ylidenemethyl]-1-aza-bicyclo[4.2.0]oct-2-ene-2-carboxylic acid sodium salt (1:1)

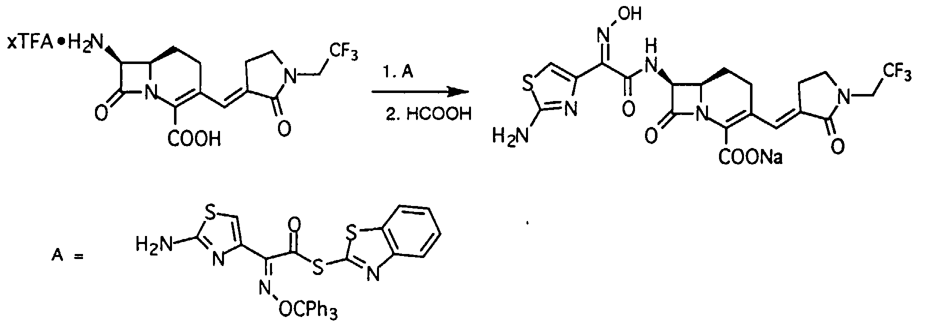

To a solution of 0.15 g (E)-(6R,7S)-7-amino-8-oxo-3-[2-oxo-1-(2,2,2-trifluoroethyl)-pyrtolidin-3-ylidenemethyl]-1-aza-bicyclo[4.2.0]oct-2-ene-2-carboxylic acid in 1.5 ml dimethyl formamide are added 0.40 g (Z)- (2-amino-thiazol-4-yl)-trityloxyimino-acetic acid benzotriazol-1-yl ester and the mixture is stirred at room temperature over night. The solvent is evaporated at 0,1 torr and the residue is taken up in 2 ml formic acid (90%) and stirred at room temperature for 2 h. The precipitate is removed by filtration and the mother liquor is evaporated at 0,1 torr. The residue is purified by chromatography on MCI-gel CHP2OP (75-150 µ) with a continuous gradient of water and acetonitrile.The product containing fractions are combined, concentrated to a volume of ca 25 ml and lyophilised. The title compound is obtained as a slightly beige lyophilisate.
Yield 0.10g (43% Th)
¹H-NMR (DMSO): inter alia 1.50-2.00 (m,2H); 3.64-3.78 (m,1H); 4.00-4.22 (m,2H); 5.35 (dd, J=8Hz,J=5Hz,1H); 6.67 (s,1H); 7.13 (s,2H); 7.50 (s,1H); 9.16 (d,J=8Hz,1H); 11.30 (s,1H) ppm.
MS (ISP): 529.0 (M-Na⁺ +2H⁺); 551.1 (M+H⁺).
IR (KBr): 1750 cm⁻¹(ν β-Lactam CO)
In an analogous manner the following compounds have been prepared

### 12.2. (6R,7S)-7-[(Z)-2-(Amino-thiazol-4-yl)-2-hydroxyimino-acetylamino]-3-[(E)-1-(4-hydroxy-phenyl)-2-oxo-pyrrolidin-3-ylidenemethyl]-8-oxo-1-aza-bicyclo[4.2.0]oct-2-ene-2-carboxylic acid sodium salt (1:1)

¹H-NMR (DMSO): inter alia 1.40-2.00 (m,2H); 3.60-3.82 (m,3H); 5.27-5.40 (m,1H); 6.67-6.83 (m,3H); 7.12 (s(br),2H); 7.40-7.60 (m,4H); 9.48 (s(br)1H); 11.25 (s(br),1H) ppm.
MS (ISP): 539.2 (M-Na⁺ +2H⁺); 561.2 (M+H⁺).
IR (KBr): 1743 cm⁻¹(ν β-Lactam CO)

### 12.3. (6R,7S)-7-[(Z)-2-(5-Amino-[1,2,4]thiadiazol-3-yl)-2-hydroxyimino-acetylamino]-3-[(E)-1-(4-hydroxy-phenyl)-2-oxo-pyrrolidin-3-ylidenemethyl]-8-oxo-1-aza-bicyclo[4.2.0]oct-2-ene-2-carboxylic acid Na salt (1:1)

¹H-NMR (DMSO): inter alia 1.50-2.00 (m,2H); 3.60-3.85 (m,3H); 5.35 (dd,J=8Hz,J=5Hz,1H); 6.79 (d,J=9Hz,2H); 7.56 (d,J=9Hz,2H); 7.58 (s,1H); 8.08 (s,2H); 9.22 (d,J=8Hz,1H); 9.84 (s,1H); 11.96 (s,1H) ppm.
MS (ISP): 540.2 (M-Na⁺ +2H⁺); 557.3 (M-Na⁺ +NH₃+2H⁺); 562.2 (M+H⁺).
IR (KBr): 1745 cm⁻¹(ν β-Lactam CO)

### 12.4. (6S,7R)-7-[(Z)-2-(Amino-thiazol-4-yl)-2-hydroxyimino-acetylamino]-3-[(E)-1-(3-nitro-phenyl)-2-oxo-pyrrolidin-2-ylidenemethyl]-8-oxo-1-aza-bicyclo[4.2.0]oct-2-ene-2-carboxylic acid sodium salt (1:1)

¹H-NMR (DMSO): inter alia 1.50-2.00 (m,2H); 3.65-4.00 (m,3H); 5.35 (dd, J=8Hz,J=5Hz,1H); 6.68 (s,1H); 7.12 (s,2H); 7.58-7.75 (m,2H); 7.92-8.00 (m,1H); 8.04-8.16 (m,1H); 8.75-8.85 (m,1H); 9.19 (d,J=8Hz,1H); 11.26 (s,1H) ppm.
MS (ISP): 568.3 (M-Na⁺ +2H⁺); 590.3 (M+H⁺).
IR (KBr): 1748 cm⁻¹(ν β-Lactam CO)

### 12.5. (6S,7R)-7-[(Z)-2-(5-Amino-[1,2,4]thiadiazol-3-yl)-2-hydroxyimino-acetylamino]-3-[(E)-1-(3-nitro-phenyl)-2-oxo-pyrrolidin-3-ylidenemethyl]-8-oxo-1-aza-bicyclo[4.2.0]oct-2-ene-2-carboxylic acid sodium salt (1:1)

¹H-NMR (DMSO): inter alia 1.40-2.00 (m,2H); 3.65-3.97 (m,3H); 5.37 (dd, J=8Hz,J=5Hz,1H); 7.60-7.75 (m,2H); 7.92-8.18 (m,4H); 9.23 (d,J=8Hz,1H); 11.89 (s,1H) ppm.
MS (ISP): 569.3 (M-Na⁺ +2H⁺); 586.3 (M-Na⁺ +NH₃+2H⁺); 591.3 (M+H⁺).
IR (KBr): 1748 cm⁻¹(ν β-Lactam CO)

### 12.6. (6R,7S)-7-[(Z)-2-(2-Amino-thiazol-4-yl)-2-hydroxyimino-acetylamino]-8-oxo-3-[(E)-2-oxo-1-pyridin-2-yl-pyrrolidin-3-ylidenemethyl]-1-aza-bicyclo[4.2.0]oct-2-ene-2-carboxylic acid Na salt (1:1)

¹H-NMR (DMSO): inter alia 1.50-2.10 (m,2H); 3.65-4.08 (m,3H); 5.38 (dd, J=8Hz,J=5Hz,1H); 6.68 (s,1H); 7.04-7.21 (m,3H); 7.64 (s,1H); 7.75-7.88 (m,1H); 8.31-8.51 (m,2H); 9.18 (d,J=8Hz,1H); 11.30 (s,1H) ppm.
MS (ISP): 524.2 (M-Na⁺ +2H⁺); 546.2 (M+H⁺).
IR (KBr): 1749 cm⁻¹(ν β-Lactam CO)

### 12.7. (6R,7S)-7-[(Z)-2-(2-Amino-thiazol-4-yl)-2-hydroxyimino-acetylamino]-8-oxo-3-[(E)-2-oxo-1-pyridin-2-yl-pyrrolidin-3-ylidenemethyl]-1-aza-bicyclo[4.2.0]oct-2-ene-2-carboxylic acid Na salt (1:1)

¹H-NMR (DMSO): inter alia 1.50-2.00 (m,2H); 3.64-3.94 (m,3H); 5.36 (dd, J=8Hz,J=5Hz,1H); 6.68 (s,1H); 7.13 (s,2H); 7.34-7.48 (m,1H); 7.60 (s,1H); 8.15-8.35 (m,2H); 8.92-9.00 (m,1H); 9.18 (d,J=8Hz,1H); 11.25 (s,1H) ppm.
MS (ISP): 524.2 (M-Na⁺ +2H⁺); 546.2 (M+H⁺); 568.3 (M+Na⁺).
IR (KBr): 1749 cm⁻¹(ν β-Lactam CO)

### 12.8. (6R,7S)-7-[(Z)-2-(2-Amino-thiazol-4-yl)-2-hydroxyimino-acetylamino]-8-oxo-3-[(E)-2-oxo-1-pyridin-4-yl-pyrrolidin-3-ylidenemethyl]-1-aza-bicyclo[4.2.0]oct-2-ene-2-carboxylic acid Na salt (1:1)

¹H-NMR (DMSO): inter alia 1.50-2.00 (m,2H); 3.60-3.87 (m,3H); 5.35 (dd, J=8Hz,J=5Hz,1H); 6.68 (s,1H); 7.13 (s,2H); 7.63 (s,1H); 7.77 (d,J=6Hz,2H); 7.99 (d,J=6Hz,2H); 9.18 (d,J=8Hz,1H); 11.27 (s,1H) ppm.
MS (ISP): 524.2 (M-Na⁺+2H⁺).
IR (KBr): 1751 cm⁻¹(ν β-Lactam CO)

### 12.9. (6R,7S)-7-[(Z)-2-(2-Amino-thiazol-4-yl)-2-hydroxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate

¹H-NMR (DMSO): inter alia 1.50-2.00 (m,2H); 3.68-3.84 (m,1H); 3.92-4.08 (m.2H); 4.15 (s,3H); 5.40 (dd, J=8Hz,J=5Hz,1H); 6.68 (s,1H); 7.13 (s,2H); 7.66 (s,1H); 8.00 (t,J= 6Hz,1H); 8.18 (d,J=6Hz,1H); 8.63 (t,J=6Hz,1H); 9.04 (d,J=6Hz,1H); 9.18 (d,J=8Hz,1H); 11.28 (s,1H) ppm.
MS (ISP): 538.4 (M+H⁺).
IR (KBr): 1756 cm⁻¹(ν β-Lactam CO)

### 12.10. (6R,7S)-7-[(Z)-2-(2-Amino-thiazol-4-yl)-2-hydroxyimino-acetylamino]-3-[(E)-1-(1-methyl-pyridin-1-ium-3-yl)-2-oxo-pyrrolidin-2-ylidenemethyl]-8-oxo-1-aza-bicyclo[4.2.0]oct-2-ene-2-carboxylate

¹H-NMR (DMSO): inter alia 1.50-2.00 (m,2H); 3.64-3.95 (m,3H); 4.38 (s,3H); 5.37 (dd, J=8Hz,J=5Hz,1H); 6.68 (s,1H); 7.14 (s,2H); 7.69 (s,1H); 8.04-8.16 (m,1H); 8.64-8.73 (m,1H); 8.82-8.91 (m,1H); 9.19 (d,J=8Hz,1H); 9.36-9.47 (m,1H); 11.27 (s,1H) ppm.
MS (ISP): 538.3 (M+H⁺).
IR (KBr): 1752 cm⁻¹(ν β-Lactam CO)

### 12.11. (6R,7S)-7-[(Z)-2-(5-Amino-[1,2,4]thiadiazol-3-yl)-hydroxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate

¹H-NMR (DMSO): inter alia 1.40-2.00 (m,2H); 3.64-3.97 (m,3H); 4.39 (s,3H); 5.35 (dd, J=8Hz,J=5Hz,1H); 7.68 (s,1H); 8.00-8.18 (m,3H); 8.62-8.73 (m,1H); 8.80-8.90 (m,1H); 9.22 (d,J=8Hz,1H); 9.35-9.44 (m,1H); 11.90 (s,1H) ppm.
MS (ISP): 539.2 (M+H⁺).
IR (KBr): 1754 cm⁻¹(ν β-Lactam CO)

### 12.12. (6R,7S)-7-[(Z)-2-(2-Amino-thiazol-4-yl)-2-hydroxyimino-acetylamino]-3-[(E)-1-(1-methyl-pyridin-1-ium-4-yl)-2-oxo-pyrrolidin-3-ylidenemethyl]-8-oxo-1-aza-bicyclo[4.2.0]oct-2-ene-2-carboxylate

¹H-NMR (DMSO): inter alia 1.50-2.00 (m,2H); 3.65-4.00 (m,3H); 4.17 (s,3H); 5.40 (dd, J=8Hz,J=5Hz,1H); 6.68 (s,1H); 7.13 (s,2H); 7.71 (s,1H); 8.28 (d,J=6Hz,2H); 8.73 (d,J=6Hz,2H); 9.19 (d,J=8Hz,1H); 11.30 (s,1H) ppm.
MS (ISP): 538.4 (M+H⁺).
IR (KBr): 1770 cm⁻¹(ν β-Lactam CO)

### 12.13. (6R,7S)-7-[(Z)-2-(2-Amino-thiazol-4-yl)-2-hydroxyimino-acetylamino]-3-[(E)-1-[1-[(3-fluoro-4-hydroxy-phenylcarbamoyl)-methyl]-pyridin-1-ium-4-yl]-2-oxo-pyrrolidin-3-ylidenemethyl)-8-oxo-1-aza-bicyclo[4.2.0]oct-2-ene-2-carboxylate, m.p.: 252°C (dec)

¹H-NMR (DMSO): inter alia 1.50-2.00 (m,2H); 3.805-4.00 (m,3H); 5.50 (m,3H); 6.71 (s,1H); 6.92 (t,1H); 7.16 (s,1H); 7.25 (d,2H); 7.62 (d,1H); 7.96 (s,1H); 8.35 (d,J=6Hz,2H); 8.82 (d,J=6Hz,2H); 9.24 (d,J=8Hz,1H); 9.74 (s,1H); 11.31 (s,1H); 11.72 (s,1H) ppm.
IR (KBr): 1753 cm⁻¹(ν β-Lactam CO)

### Example 13

### Synthesis of (6R,7S)-7-[(E)-2-(2-Amino-thiazol-4-yl)-2-hydroxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate

By using trifluoroacetic acid and triethyl silane instead of formic acid as discribed in Example 12 in the deprotection step the (E) oxime isomer is obtained.
¹H-NMR (DMSO): inter alia 1.50-1.90 (m,2H); 3.60-3.95 (m,3H); 4.40 (s,3H); 5.37 (dd, J=8Hz,J=5Hz,1H); 7.20 (s,2H); 7.53 (s,1H); 7.66 (s,1H); 8.03-8.16 (m,1H); 8.64-8.72 (m,1H); 8.82-8.91 (m,1H); 9.16 (d,J=8Hz,1H); 9.37-9.43 (m,1H); 12.57 (s,1H) ppm.

### Example 14

### 14.1. Synthesis of (6R,7S)-7-[(Z)-2-Acetoxyimino-2-(2-amino-thiazol-4-yl)-acetylamino]-8-oxo-3-[(E)-2-oxo-1-(2,2,2-trifluoro-ethyl)-pyrrolidin-3-ylidenemethyl]-1-aza-bicyclo[4.2.0]oct-2-ene-2-carboxylic acid sodium salt (1:1)

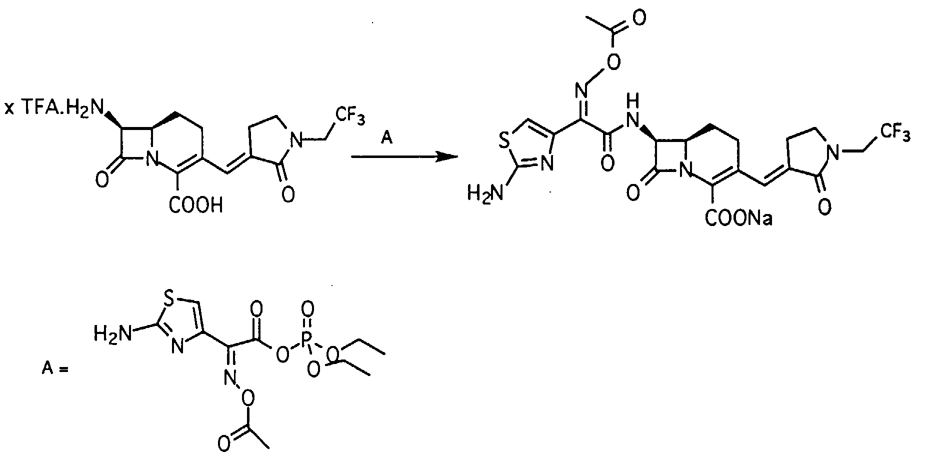

To a solution of 1.90 g (E)-(6R,7S)-7-amino-8-oxo-3-[2-oxo-1-(2,2,2-trifluoroethyl)-pyrrolidin-3-ylidenemethyl]-1-aza-bicyclo[4.2.0]oct-2-ene-2-carboxylic acid and 1,3-bis(trimethylsilyl)urea in 20 ml N,N-dimethylformamide are added 2.42 g (Z)-(2-amino-thiazol-4-yl)-acetoxyimino-acetic acid diethoxythiophosphorylester and the mixture is stirred at room temperature for 2 h. The solvent is evaporated at 0,1 torr. The residue is taken up in ethylacetate and the resulting solid is collected by filtration. The raw product is optionally purified by chromatography on MCI-gel CHP2OP (75-150 µ) with a continuous gradient of water and acetonitrile.The product containing fractions are combined, concentrated to a volume of ca 25 ml and lyophilised. The title compound is obtained as a slightly beige lyophilisate.
Yield 1.00 g (after chromatography)
¹H-NMR (DMSO): inter alia 1.50-1.90 (m,2H); 2.15 (s,3H); 3.70-3.85 (m,1H); 4.00-4.25 (m,2H); 5.35 (dd, J=8Hz,J=5Hz,1H); 7.09 (s,1H); 7.37 (s,2H); 7.50 (s,1H); 9.56 (d,J=8Hz,1H)ppm.
MS (ISP): 571.3 (M+H⁺); 593.3 (M+Na⁺).
IR (KBr): 1756 cm⁻¹(ν β-Lactam CO)
In a similar manner the following compounds have been prepared from their corresponding 7-amino-carbacephem precursors

### 14.2. (6R,7S)-7-[(Z)-2-Acetoxyimino-2-(2-amino-thiazol-3-yl)acetylamino]-3-[(E)-1-(4-hydroxy-phenyl)-2-oxo-pyrrolidin-3-ylidenemethyl]-8-oxo-1-aza-bicyclo[4.2.0]oct-2-ene-2-carboxylic acid

¹H-NMR (DMSO): inter alia 2.16 (s,3H); 3.70-4.00 (m,3H); 5.54 (dd, J=8Hz,J=5Hz,1H); 6.77 (d,J=9Hz,2H); 7.11 (s,1H); 7.37 (s,2H); 7.44 (s,1H); 7.55 (d,J=9Hz,2H); 9.39 (s,1H); 9.58 (d,J=8Hz,1H)ppm.
MS (ISP): 581.3 (M+H⁺); 603.3 (M+Na⁺).
IR (KBr): 1760 cm⁻¹(ν β-Lactam CO)

### 14.3. (6R,7S)-7-[(Z)-2-Acetoxyimino-2-(5-amino-thiazol-3-yl)-acetylamino]-3-[(E)-1-(3-nitro-phenyl)-2-oxo-pyrrolidin-3-ylidenemethyl]-8-oxo-1-aza-bicyclo[4.2.0]oct-2-ene-2-carboxylic acid

¹H-NMR (DMSO): inter alia 2.17 (s,3H); 3.80-4.04 (m,3H); 5.60 (dd, J=8Hz,J=5Hz,1H); 7.12 (s,1H); 7.37 (s,2H); 7.56 (s,1H); 7.68-7.77 (m,1H); 7.99-8.14 (m,2H); 8.83-8.92 (m,1H); 9.60 (d,J=8Hz,1H)ppm.
MS (ISP): 610.3 (M+H⁺); 627.3 (M+Na⁺).
IR (KBr): 1765 cm⁻¹(ν β-Lactam CO)

### 14.4. (6R,7S)-7-[(Z)-2-Acetoxyimino-2-(2-amino-thiazol-4-yl)-acetylamino]-8-oxo-3-[(Z)-2-oxo-1-pyridin-2-yl-pyrrolidin-3-ylidenemethyl]-1-aza-bicyclo[4.2.0]oct-2-ene-2-carboxylic acid Na salt (1:1)

¹H-NMR (DMSO): inter alia 2.16 (s,3H); 3.75-4.10 (m,3H); 5.36 (dd, J=8Hz,J=5Hz,1H); 7.06-7.16 (m,2H); 7.37 (s,2H); 7.62 (s,1H); 7.75-7.88 (m,1H); 8.34-8.50 (m,2H); 9.58 (d,J=8Hz,1H)ppm.
MS (ISP): 566.4 (M-Na⁺ +2H⁺); 588.3 (M+H⁺).
IR (KBr): 1756 cm⁻¹(ν β-Lactam CO)

### 14.5. (6R,7S)-7-[(Z)-2-Acetoxyimino-2-(2-amino-thiazol-4-yl)-acetylamino]-8-oxo-3-[(Z)-2-oxo-1-pyridin-3-yl-pyrrolidin-3-ylidenemethyl]-1-aza-bicyclo[4.2.0]oct-2-ene-2-carboxylic acid.

MS (ISP): 566.5 (M+H⁺).
IR (KBr): 1764 cm⁻¹(ν β-Lactam CO)

### 14.6. (6R,7S)-7-[(Z)-2-Acetoxyimino-2-(2-amino-thiazol-4-yl)-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

¹H-NMR (DMSO): inter alia 2.16 (s,3H); 3.67-3.86 (m,3H); 4.39 (s,3H); 5.37 (dd, J=8Hz,J=5Hz,1H); 7.10 (s,1H); 7.38 (s,2H); 7.66 (s,1H); 8.04-8.16 (m,1H); 8.64-8.73 (m,1H); 8.85-8.94 (m,1H); 9.35-9.44 (m,1H); 9.60 (d,J=8Hz,1H)ppm.
MS (ISP): 580.3 (M+H⁺); 602.3 (M+Na⁺).
IR (KBr): 1757 cm⁻¹(ν β-Lactam CO)

### 14.7. (6R,7S)-7-[(Z)-2-Acetoxyimino-2-(2-amino-thiazol-4-yl)-acetylamino]-3-[(E)-1-(1-benzyl-piperidin-4-yl)-2-oxo-pyrrolidin-3-ylidenemethyl]-8-oxo-1-aza-bicyclo[4.2.0]oct-2-ene-2-carboxylic acid

MS (ISP): 662.4 (M+H)⁺; 684.3 (M+Na)⁺.
IR (KBr): 1757 cm⁻¹(ν β-Lactam CO)

### 14.8. (6R,7S)-7-[(Z)-2-Acetoxyimino-2-(2-amino-thiazol-4-yl)-acetylamino]-8-oxo-3-[(E)-2-oxo-1-pyridin-2-yl-pyrrolidin-3-ylidenemethyl]-1-aza-bicyclo-[4.2.0]oct-2-ene-2-carboxylic acid

MS (ISP): 572.3 (M+H)⁺; 594.3 (M+Na)⁺.
IR (KBr): 1765 cm⁻¹(ν β-Lactam CO)

### Example 15

### Synthesis of (6R,7S)-7-[(Z)-2-Acetoxyimino-2-(2-amino-thiazolo-4-yl)-acetylamino]-3-[(E)-1-(1-carbamoylmethyl-pyridin-1-ium-3-yl)-2-oxo-pyrrolidin-3-ylidenemethyl]-8-oxo-1-aza-bicyclo[4.2.0]oct-2-ene-2-carboxylate

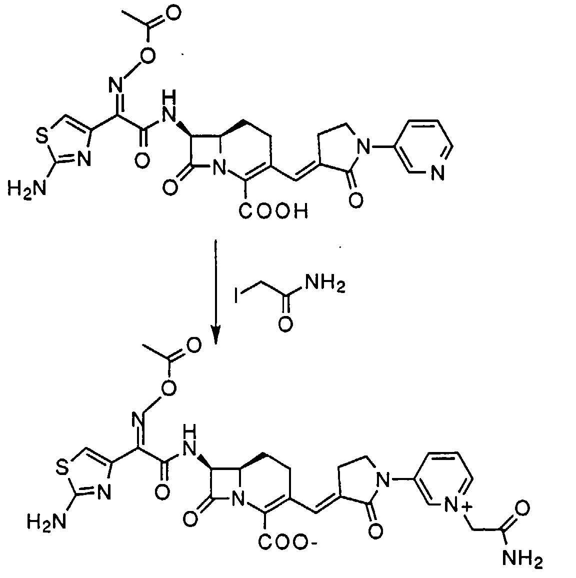

To a solution of 0.17 g (6R,7S)-7-[(Z)-2-acetoxyimino-2-(2-amino-thiazol-4-yl)-acetylamino]-8-oxo-3-[(Z)-2-oxo-1-pyridin-3-yl-pyrrolidin-3-ylidenemethyl]-1-aza-bicyclo[4.2.0]oct-2-ene-2-carboxylic acid and 0.305 g 1,3-bis(trimethylsilyl)urea in 3 ml N,N-dimethylformamide are added 0.055 g iodoacetamide and the mixture is stirred at roomtemperature for 72h. The solvent is evaporated at 0,1 torr and the residue is taken up in water. The precipitate is collected by filtration and dried.
yield: 0.08 g
¹H-NMR (DMSO): inter alia 2.17 (s,3H); 3.75-4.05 (m,3H); 5.46 (s,2H); 5.52 (dd, J=8Hz,J=5Hz,1H); 7.12 (s,1H); 7.39 (s,2H); 7.62 (s,1H); 7.72 (s,1H); 8.07 (s,1H); 8.10-8.26 (m,1H); 8.67-8.75 (m,1H); 8.87-8.98 (m,1H); 9.43-9.54 (m,1H); 9.60 (d,J=8Hz,1H)ppm.
MS (ISP): 623.5 (M+H⁺).
IR (KBr): 1761 cm⁻¹(ν β-Lactam CO)

### Example 16

### 16.1. Synthesis of (6R,7S)-7-[(Z)-2-(2-Amino-thiazol-4-yl)-2-methoxyimino-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

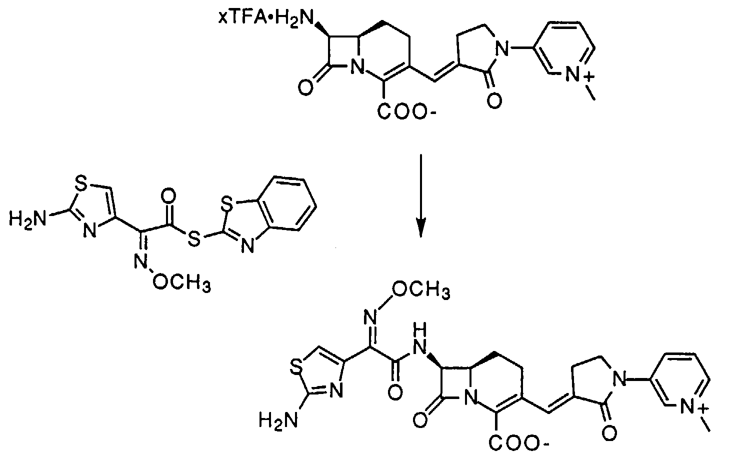

To a suspension of 0.16 g (E)-(6R,7S)-7-amino-3-[1-(1-methyl-pyridin-1-ium-3-yl)-2-oxo-pyrrolidin-3-ylidenemethyl]-8-oxo-1-aza-bicyclo[4.2.0]oct-2-ene-2-carboxylate trifluoroacetate (1:1) in 1.6 ml N,N-dimethylacetamide are added 0.15 g (Z)-(2-amino-thiazol-4-yl)-methoxyimino-thioacetic acid S-benzothiazol-2-yl ester and the mixture is stirred for 1h at room temperature. The solvent is evaporated at 0,1 torr and the residue is distributed between water and ethyl acetate. The aqueous phase is purified by chromatography on MCI-gel CHP2OP (75-150 µ) with a continuous gradient of water and acetonitrile. The product containing fractions are combined, concentrated to a volume of ca 25 ml and lyophilised. The title compound is obtained as a yellow lyophilisate.
yield: 0.07 g
¹H-NMR (DMSO): inter alia 1.40-2.00 (m,2H); 3.64-3.96 (m,3H) superimposed by 3.85 (s,3H); 4.40 (s,3H); 5.36 (dd, J=8Hz,J=5Hz,1H); 6.76 (s,1H); 7.23 (s,2H); 7.65 (s,1H); 8.04-8.15 (m,1H); 8.64-8.75 (m,1H); 8.85-8.96 (m,1H); 9.32 (d,J=8Hz,1H); 9.35-9.45 (m,1H); ppm.
MS (ISP): 552.3 (M+H⁺); 574.4 (M+Na⁺).
IR (KBr): 1753 cm⁻¹(ν β-Lactam CO)
In a similar manner the following compounds have been prepared, using the appropriately substituted (2-Amino-thiazol-4-yl)-oxyimino-thioacetic acid S-benzothiazol-2-yl ester.

### 16.2. (6R,7S)-7-[(Z)-2-(2-Amino-thiazol-4-yl)-2-(1-carbamoyl-1-methylethoxyimino)-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

¹H-NMR (DMSO): inter alia 1.36 (s,3h); 1.38 (s,3H); 1.40-2.00 (m,2H); 3.69-3.97 (m,3H); 4.39 (s,3H); 5.36 (dd, J=8Hz,J=5Hz,1H); 6.82 (s,1H); 6.93 (s,1H); 7.23 (s,1H); 7.34 (s,2H); 7.67 (s,1H); 8.05-8.15 (m,1H); 8.65-8.74 (m,1H); 8.85-8.95 (m,1H); 9.35-9.44 (m,1H); 9.48 (d,J=8Hz,1H) ppm.
MS (ISP): 623.4 (M+H⁺).
IR (KBr): 1757 cm⁻¹(ν β-Lactam CO)

### 16.3. (6R,7S)-7-[(Z)-2-(2-Amino-thiazol-4-yl)-2-tert-butoxycarbonylmethoxyimino-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

¹H-NMR (DMSO): inter alia 1.44 (s,9H); 3.64-3.95 (m,3H); 4.39 (s,3H); 4.56 (s,2H); 5.36 (dd, J=8Hz,J=5Hz,1H); 6.78 (s,1H); 7.24 (s,2H); 7.67 (s,1H); 8.04-8.16 (m,1H); 8.64-8.74 (m,1H); 8.81-8.90 (m,1H); 9.31 (d,J=8Hz,1H); 9.37-9.45 (m, 1H); ppm.
MS (ISP): 652.4 (M+H⁺).
IR (KBr): 1751 cm⁻¹(ν β-Lactam CO)

### Example 17

### 17.1. (6R,7S)-7-[(Z)-2-(2-Amino-thiazol-4-yl)-2-(1-carboxy-1-methoxyimino)-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate Na salt (1:1).

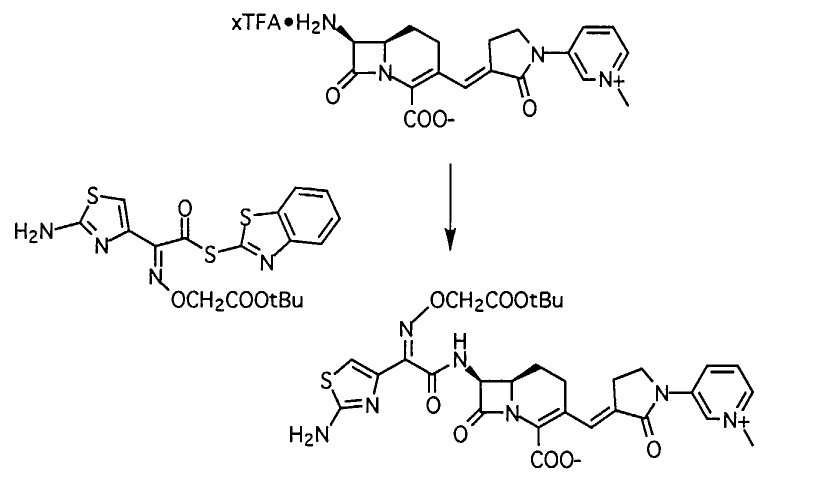

To a suspension of 0.50 g (E)-(6R,7S)-7-amino-3-[1-(1-methyl-pyridin-1-ium-3-yl)-2-oxo-pyrrolidin-3-ylidenemethyl]-8-oxo-1-aza-bicyclo[4.2.0]oct-2-ene-2-carboxylate trifluoroacetate (1:1) in 2.5 ml N,N-dimethylacetamide are added 0.50 g (Z)-[(2-amino-thiazol-4-yl)-(benzothiazol-2-ylsulfanylcarbonyl)-methyleneaminooxy]-acetic acid tert-butyl ester and the mixture is stirred for 2.5 h at room temperature. The solvent is evaporated at 0,1 torr and the residue is treated with 5 ml trifluoroacetic acid at room temperature for 1h. The solvent is evaporated and the residue is distributed between water and ethyl acetate at pH 7.00. The aqueous phase is purified by chromatography on MCI-gel CHP2OP (75-150 µ) with a continuous gradient of water and acetonitrile. The product containing fractions are combined, concentrated to a volume of ca 25 ml and lyophilised. The title compound is obtained as a yellow lyophilisate.
Yield : 0.29 g
¹H-NMR (DMSO): inter alia 3.58-3.90 (m,3H); 4.14-4.31 (m,2H); 4.41 (s,3H); 5.45 (dd, J=8Hz,J=5Hz,1H); 6.86 (s,1H); 7.19 (s,2H); 7.49 (s,1H); 8.02-8.12 (m,1H); 8.81-8.91 (m,1H); 9.00-9.08 (m,1H); 9.40-9.49 (m,1H); 11.46 (d,J=8Hz,1H) ppm.
MS (ISP): 596.4 (M-Na⁺+2H⁺).
IR (KBr): 1751 cm⁻¹(ν β-Lactam CO)

| | | | |
|---|---|---|---|
| MA (calc.for C₂₆H₂₄N₇O₈SNa + 11.29 % water: | C:50.57; | H:3.92; | N:15.88; |
| found: | C:50.81; | H:3.90; | N:15.97 (%) |

In an analogous manner the following compounds were prepared

### 17.2. (6R,7S)-7-[(Z)-2-(2-Amino-thiazol-4-yl)-2-(1-carboxy-1-methylethoxyimino)-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate Na salt (1:1) has been prepared.

¹H-NMR (DMSO): inter alia 1.37 (s,3H); 1.43 (s,3H); 3.58-3.88 (m,3H); 4.41 (s,3H); 5.50 (dd, J=8Hz,J=5Hz,1H); 6.76 (s,1H); 7.18 (s,2H); 7.45 (s,1H); 8.00-8.11 (m,1H); 8.99-9.10 (m,2H); 9.15-9.25 (m,1H); 11.60 (d(br),J=8Hz,1H) ppm.
MS (ISP): 624.3 (M-Na⁺+2H⁺).
IR (KBr): 1752 cm⁻¹(ν β-Lactam CO)

| | | | |
|---|---|---|---|
| MA (calc.for C₂₈H₂₈N₇O₈SNa + 10.5 % water : | C:52.09; | H:4.37; | N:15.19; |
| found: | C:52.05; | | N:15.17(%) |

### Example 18

### 18.1. Synthesis of (6R,7S)-7-[(Z)-2-(2-Amino-thiazol-4-yl)-2-(2-fluoroethoxyimino)-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

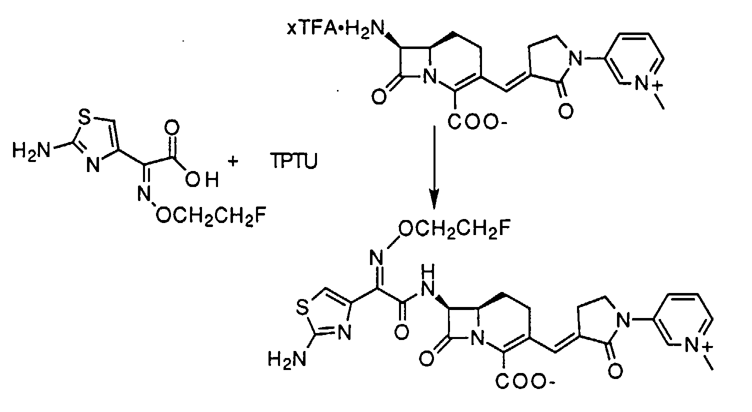

To a solution of 0.23 g (Z)-(2-amino-thiazol-4-yl)-(2-fluoro-ethoxyimino)-acetic acid and 0.15 ml triethylamine in 5.0 ml N,N-dimethylacetamide are added 0.30 g 1,1,3,3-tetramethyl-2-[2-oxo-1(2H)-pyridyl]uronium tetrafluoroborate (1:1) (TPTU) and the mixture is stirred for 10 min at room temperature. To this mixture 0.50 g (E)-(6R,7S)-7-Amino-3-[1-(1-methyl-pyridin-1-ium-3-yl)-2-oxo-pyrrolidin-3-ylidenemethyl]8-oxo-1-aza-bicyclo[4.2.0]oct-2-ene-2-carboxylate trifluoroacetate (1:1) is added and strirting is continued for 4 h at room temperature. Another 0.30 ml of triethylamine is added and the mixture is stirred for 1 h. The solvent is evaporated and the residue is distributed between water and ethyl acetate at pH 7.00. The aqueous phase is purified by chromatography on MCI-gel CHP2OP (75-150 µ) with a continuous gradient of water and acetonitrile.The product containing fractions are combined, concentrated to a volume of ca 25 ml and lyophilised. The title compound is obtained as a yellow lyophilisate.
Yield : 0.17 g
¹H-NMR (DMSO): inter alia 1.50-2.00 (m,2H); 3.64-3.98 (m,3H); 4.18-4.80 (m,4H) superimposed by 4.39 (s,3H); 5.34 (dd, J=8Hz,J=5Hz,1H); 6.79 (s,1H); 7.25 (s,2H); 7.66 (s,1H); 8.03-8.15 (m,1H); 8.65-8.75 (m,1H); 8.84-8.94 (m,1H); 9.36 (d,J=8Hx,1H) superimposed by 9.36-9.44 (m,1H) ppm.
MS (ISP): 584.4 (M+H⁺).
IR (KBr): 1754 cm⁻¹(ν β-Lactam CO)

| | | | |
|---|---|---|---|
| MA (calc.for C₂₆H₂₆N₇FO₆S + 7.35 % water and 1.3% ashes : | C:53.51; | H:4.49; | N:16.18; |
| found: | C:53.33; | H:4.46; | N:16.38 (%) |

## Claims

1. 1-carba-(dethia)-cephalosporin derivatives of the general formula I 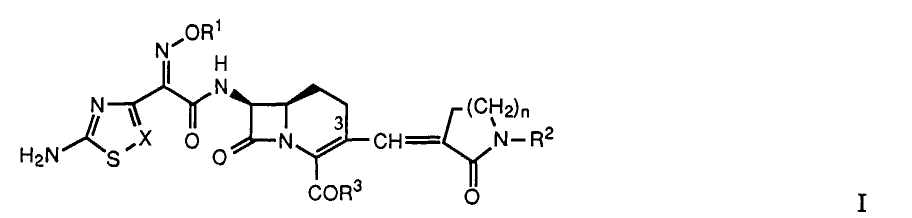 wherein
R¹ is hydrogen, optionally fluoro substituted lower alkyl, aralkyl, cycloalkyl, -COR⁴ or -C(R⁵R⁶)CO₂R⁷ -C(R⁵R⁶)CONHR⁷; where R⁵ and R⁶ are each independently hydrogen or lower alkyl, or R⁵ and R⁶ taken together form a cycloalkyl group; R⁴ is hydrogen or lower alkyl and R⁷ is hydrogen, lower alkyl, lower alkenyl or a carboxylic acid protecting group;
R² is hydrogen, hydroxy, lower alkyl-Qₘ, cycloalkyl, lower alkoxy, lower alkenyl, cycloalkenyl, lower alkynyl, aralkyl-Qₘ, aryl-Qₘ, aryloxy, aralkoxy, a heterocyclic ring or heterocyclyl lower alkyl, the lower alkyl, cycloalkyl, lower alkoxy, lower alkenyl, cycloalkenyl, lower alkynyl, aralkyl, aryl, aryloxy, aralkoxy and the heterocyclic ring being unsubstituted or substituted with at least one group selected from carboxy, amino, nitro, cyano, optionally fluoro substituted lower alkyl, lower alkoxy, hydroxy, halogen, -COR⁶, -C(R⁵R⁶)CO₂R⁷, -C(R⁵R⁶)CONR⁵R⁸, -CONR⁵R⁶, -N(R⁶)COOR¹⁰, R⁶OCO- or R⁶COO- where R⁵ and R⁶ are hydrogen or lower alkyl; R⁷ is hydrogen, lower alkyl, lower alkenyl or a carboxylic acid protecting group; R⁸ is hydrogen, lower alkyl or optionally substituted phenyl; R¹⁰ is lower alkyl, lower alkenyl or a carboxylic acid protecting group;
Q is -CHR-, -CO- or -SO₂-;
R is hydrogen or lower alkyl
R³ is hydroxy, lower-alkoxy, or -O⁻, when R² has a positive charge, or -OM and M represents an alkali metal;
m is 0 or 1;
n is 0, 1 or 2;
X is CH or N
as well as readily hydrolysable esters thereof, pharmaceutically acceptable salts of said compounds and hydrates of the compounds of formula I and of their esters and salts.

2. Compounds of claim 1 with the 3-substituent in the E-form and the 7-substituent in Z-form.

3. Compounds of claims 1 or 2, wherein n is 0 or 1.

4. Compounds of any one of claims 1 to 3, wherein R¹ is hydrogen, optionally fluoro substituted lower alkyl, cycloalkyl, -COR⁴, -C(R⁵R⁶)CO₂R⁷ or -C(R⁵R⁶)CONHR⁷ and R⁴, R⁵, R⁶ and R⁷ are as defined in claim 1.

5. Compounds of any one of claims 1 to 4, wherein R² is lower alkyl-Q, where Q is -CHR- and R is hydrogen; or R² is optionally fluoro substituted lower alkyl, aryl or a heterocyclic ring, the lower alkyl, cycloalkyl, aryl, and the heterocyclic ring being unsubstituted or substituted with at least one group selected from nitro, optionally fluoro substituted lower alkyl, hydroxy or halogen.

6. Compounds of any one of claims 1 to 5, wherein R² is pyridine-2-yl, -3-yl or -4-yl, 1-methylpridinium-2-yl, -3-yl or -4-yl, 1-carbamoylmethylpridinium-2-yl, -3-yl or -4-yl, 1-(N-phenylcarbamoylmethyl)-pyridinium-2-yl, -3-yl or -4-yl or 1-[N-(3-fluoro-4-hydroxyphenyl)carbamoylmethyl]pyridinium-2-yl, -3-yl or -4-yl,.

7. The compound (6R,7S)-7-[(Z)-2-Acetoxyimino-2-(2-amino-thiazolo-4-yl)-acetylamino]-3-[(E)-1-(1-carbamoylmethyl-pyridin-1-ium-3-yl)-2-oxo-pyrrolidin-3-ylidenemethyl]-8-oxo-1-aza-bicyclo[4.2.0]oct-2-ene-2-carboxylate

8. (6R,7S)-7-[(Z)-2-(2-Amino-thiazol-4-yl)-2-hydroxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate

9. (6R,7S)-7-[(Z)-2-(2-Amino-thiazol-4-yl)-2-hydroxyimino-acetylamino]-8-oxo-3-[(E)-2-oxo-1-(2,2,2-trifluoro-ethyl)-pyrrolidin-3-ylidenemethyl]-1-aza-bicyclo[4.2.0]oct-2-ene-2-carboxylic acid sodium salt (1:1)

10. (6R,7S)-7-[(Z)-2-(Amino-thiazol-4-yl)-2-hydroxyimino-acetylamino]-3-[(E)-1-(4-hydroxy-phenyl)-2-oxo-pyrrolidin-3-ylidenemethyl]-8-oxo-1-aza-bicyclo[4.2.0]oct-2-ene-2-carboxylic acid sodium salt (1:1)

11. (6R,7S)-7-[(Z)-2-Acetoxyimino-2-(2-amino-thiazol-4-yl)-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

12. (6R,7S)-7-[(Z)-2-(2-Amino-thiazol-4-yl)-2-methoxyimino-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

13. (6R,7S)-7-[(Z)-2-(2-Amino-thiazol-4-yl)-2-cyclopentyloxyimino-acetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

14. (6R,7S)-7-[(Z)-2-(5-Amino-[1,2,4]thiadiazol-3-yl)-hydroxyimino-acetylamino]-3-[(E)-1-(1-methyl-pyridin-1-ium-3-yl)-2-oxo-pyrtolidin-3-ylidenemethyl]-8-oxo-1-aza-bicyclo[4.2.0]oct-2-ene-2-carboxylate

15. (6R,7S)-7-[(Z)-2-(5-Amino-[1,2,4]thiadiazol-3-yl)-cyclopentyloximinoacetylamino]-3-[(E)-1-(1-methyl-pyridin-1-ium-3-yl)-2-oxo-pyrroliden-3-ylidenemethyl]-8-oxo-1-aza-bicyclo[4.2.0]oct-2-ene-2-carboxylate

16. (6R,7S)-7-[(Z)-2-(2-Amino-thiazol-4-yl)-2-carboxymethoxyimino-acetylamino]-3-[(E)-1-(1-methyl-pyridin-1-ium-3-yl)-2-oxo-pyrrolidin-3-ylidenemethyl]-8-oxo-1-aza-bicyclo[4.2.0]oct-2-ene-2-carboxylate Na salt (1:1)

17. (6R,7S)-7-[(Z)-2-(2-Amino-thiazol-4-yl)-2-cyclopentyloxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate

18. (6R,7S)-7-[(Z)-2-(2-Amino-thiazol-4-yl)-2-hydroxyimino-acetylamino]-3-[(E)-1-(1-methyl-pyridin-1-ium-2-yl)-2-oxo-pyrrolidin-3-ylidenemethyl]-8-oxo-1-aza-bicyclo[4.2.0]oct-2-ene-2-carboxylate

19. (6R,7S)-7-[(Z)-2-(2-Amino-thiazol-4-yl)-2-hydroxyimino-acetylamino]-3-[(E)-1-(1-methyl-pyridin-1-ium-4-yl)-2-oxo-pyrrolidin-3-ylidenemethyl]-8-oxo-1-aza-bicyclo[4.2.0]oct-2-ene-2-carboxylate

20. (6R,7S)-7-[(Z)-2-(2-Amino-thiazol-4-yl)-2-cyclopentyloxyimino-acetylaminol-3-[(E)-1-(1-methyl-pyridin-1-ium-4-yl)-2-oxo-pyrrolidin-3-ylidenemethyl]-8-oxo-1-aza-bicyclo[4.2.0]oct-2-ene-2-carboxylate

21. Compounds of the formula II 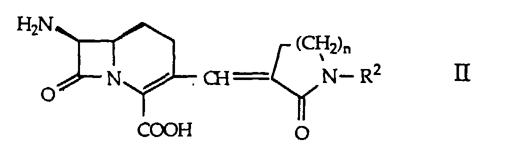 in which R² and n are defined above,
or esters or salts thereof.

22. A pharmaceutical preparation containing a compound according to any one of claims 1-20, particularly for the treatment and prophylaxis of infectious diseases.

23. Process for the manufacture of the compounds according to any one of claims 1-20, which process comprises
(a) treating a compound having the formula II 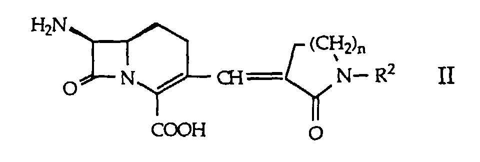 in which R² and n are defined above,
or an ester or salt thereof, with a carboxylic acid of the general formula III 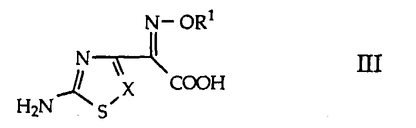 in which R¹ and X are defined above, or a reactive functional derivative thereof, or
(b) cleaving off the amino, hydroxy and/or carboxy protecting group in a compound having the formula IV 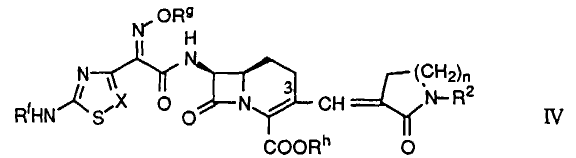 in which R² is defined above, R^{f} is hydrogen or an amino protecting group, R^{g} is hydrogen or a hydroxy protecting group, R^{h} is hydrogen or a carboxy protecting group, provided that at least one of R^{f}, R^{g} and R^{h} is a corresponding protecting group or a salt thereof, or by
(c) alkylation of a compound of formula 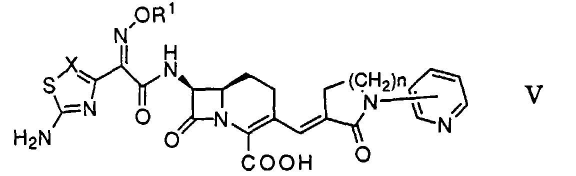 wherein R¹, X and n are as defined above,
with a alkylating agent such a methyliodide, dimethylsulfate or trimethyloxonium tetrafluoroborate or bromo- or iodoacetamide, or
(d) for the manufacture of a readily hydrolysable ester of a compound of formula I subjecting a carboxylic acid of formula I to a corresponding esterification, or
(e) for the manufacture of salts or hydrates of a compound of formula I or hydrates of said salts converting a compound of formula I into a salt or hydrate or into a hydrate of said salts.

24. Manufacture of compounds of formula I according to claim 1 wherein R² is 1-methyl-pyridinium which comprises protection of the sensitive groups in compound of formula I wherein R² is a pyridin ring, quaternisation of the pyridin ring and deprotection of the sensitive groups.

25. Compounds according to any one of claims 1-20, whenever prepared according to the process claimed in claim 23 or by an obvious chemical equivalent thereof.

26. Compounds as in any one of claims 1-20 as pharmaceutically active substances for the treatment and prophylaxis of illnesses, particularly for the treatment and prophylaxis of infectious diseases.

27. The use of the compounds according to any one of claims 1-20 in the treatment and prophylaxis of illnesses, particularly in the treatment and prophylaxis of infectious diseases.

28. The use of the compounds according to any one of claims 1-20 for the manufacture of medicaments for the treatment and prophylaxis of infectious diseases.

29. The novel compounds, formulations, processes and methods substantially as described herein.
